(19) Europäisches Patentamt
European Patent Office
Office européen des brevets

(11) Veröffentlichungsnummer: **0 015 230 B1**

# (12) EUROPÄISCHE PATENTSCHRIFT

(45) Veröffentlichungstag der Patentschrift: **23.02.83**

(51) Int. Cl.³: **C 08 K 5/34**, C 09 D 3/80

(21) Anmeldenummer: **80810017.6**

(22) Anmeldetag: **21.01.80**

(54) **Stabilisiertes organisches Polymer enthaltend als Lichtstabilisator ein 2-(2-Hydroxy-3,5-di-tert-octylphenyl)-2H-benzotriazol.**

(30) Priorität: **25.01.79 US 6391**
**17.12.79 US 100400**

(43) Veröffentlichungstag der Anmeldung: **03.09.80 Patentblatt 80/18**

(45) Bekanntmachung des Hinweises auf die Patenterteilung: **23.02.83 Patentblatt 83/8**

(84) Benannte Vertragsstaaten: **DE FR GB IT NL**

(56) Entgegenhaltungen:
**FR-A-2 364 947**
**US-A-3 230 194**
**US-A-4 041 044**
**Chemical Reviews, 46, p. 55/1950**

(73) Patentinhaber: **CIBA-GEIGY AG, Patentabteilung Postfach, CH-4002 Basel (CH)**

(72) Erfinder: **Dexter, Martin, 416 Cedar Drive West, Briarcliff Manor, N.Y. 10510 (US)**
Erfinder: **Winter, Roland A.E., 23 Banksville Road, Amonk, New York 10504 (US)**

**EP 0 015 230 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Stabilisiertes organisches Polymer enthaltend als Lichtstabilisator ein 2-[2-Hydroxy-3,5-di-tert.-octylphenyl]-2H-benzotriazol

UV-Absorber vom Typ der o-Hydroxyphenyl-2H-benzotriazolen geniessen seit langem ein erhebliches wirtschaftliches Interesse als Lichtstabilisatoren für organisches Material.

Solche Verbindungen, deren Herstellung und Verwendung dieser wertvollen 2-Aryl-2H-benzotriazolen sind z. B. aus den folgenden Patentschriften bekannt: US 3 004 896, US 3 055 896, US 3 072 585, US 3 074 910, US 3 189 615 und US 3 230 194. Diese Stabilisatoren zeigen jedoch eine schlechte Verträglichkeit in gewissen Substraten. Sie neigen stark zum Ausschwitzen, Sublimieren und/oder zum Verflüchtigen während der Verarbeitung zu Folien, Filmen, Fasern usw. bei hohen Temperaturen. Auf diese Weise geht ein unangemessen hoher Teil des Benzotriazolstabilisators verloren, insbesondere bei extrem dünnen Filmen und Überzügen. Dies gilt auch, wenn die Filme oder Überzüge während der Verwendung erhöhter Temperatur ausgesetzt sind.

Es sind Versuche unternommen worden, durch strukturelle Abwandlung des Benzotriazols, die Verträglichkeit zu verbessern und den Stabilisatorschwund zu reduzieren.

US-PS 3 230 194 beschreibt Benzotriazole, substituiert mit einer langkettigen Alkyl-Gruppe anstelle von Methyl-Gruppe, insbesondere 2-[2-Hydroxy-5-tert.-octylphenyl]-2H-benzotriazol, welche ausgezeichnete Verträglichkeit und stabilisierende Wirksamkeit zeigt in Polyäthylen.

Wieder andere Versuche, welche unternommen werden, um die Verträglichkeit der Aryl-2H-benzotriazole mit dem polymeren Substrat zu verbessern und den Stabilisatorschwund durch Verflüchtigung der Substanzen während der Verarbeitung oder Verwendung herabzusetzen, zielten auf die Substitution des Phenylringes mit Aralkylgruppen, wie Benzyl, α-Methylbenzyl oder α,α-Dimethylbenzyl ab. Solche Verbindungen sind in US 4 127 586 und in JP Kokai 158 588/75 beschrieben.

Verbindungen, wie z. B. 2-(2-Hydroxy-3,5-di-tert.-octylphenyl)-2H-benzotriazol, zeigen eine ausgezeichnete Verträglichkeit und Löslichkeit in einer Vielzahl von Polymeren und gleichzeitig einen überraschend geringen Verlust in stabilisierten Zusammensetzungen während der Hochtemperatur-Verarbeitung oder in deren Endverwendung. Die stabilisierten Mischungen, welche als Filme und Überzüge ständigen Witterungs- und Lichteinwirkungen ausgesetzt sind, werden wirksamer geschützt als durch andere, bekannte 2-Aryl-2H-benzotriazole, welche strukturell den genannten Verbindungen nahestehen.

US-Patent 3 487 453 beschreibt ein anfärbbares stabilisiertes Polymergemisch, welches Polypropylen, Polyätherester, zwei phenolische Antioxidantien, einen Thiosynergisten und 2-(2-Hydroxy-3,5-dioctylphenyl)-2,1,3-benzotriazol enthält. Die exakte chemische Struktur der «Dioctyl»-Substituenten ist jedoch nicht identifiziert. Der Einfluss und die Wirksamkeit des Benzotriazols kann in dem komplexen Gemisch von Stabilisatoren und Polymeren ebenfalls nicht erkannt werden.

In US-PS 4 041 044 ist ein verbessertes Verfahren zur Herstellung der 2-Aryl-2H-benzotriazole beschrieben, und eine Anzahl von Phenolen als Ausgangsmaterialien angegeben, von denen etwa zwölf Phenole bevorzugt werden.

Obwohl 2,4-Di-tert.-octylphenol unter den brauchbaren Phenolen aufgezählt ist, wird weder die Herstellung von 2-(2-Hydroxy-3,5-di-tert.-octylphenyl)-2H-benzotriazol noch von 5-Chlor-2-(2-hydroxy-3,5-di-tert.-octylphenyl)-2H-benzotriazol angegeben oder als Beispiel genannt. Die ausgezeichneten Eigenschaften der Verbindungen der Formel I im Vergleich mit bekannten Benzotriazolen ist unter den unzähligen möglichen Verbindungen, welche diese Patentschrift offenbart, nicht erkannt worden.

Die Erfindung betrifft stabilisiertes organisches Polymer, enthaltend als Stabilisator eine Verbindung der Formel I

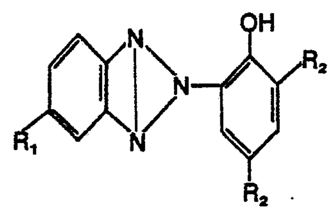

(I),

worin $R_1$ Wasserstoff oder Chlor ist und $R_2$ tert.-Octyl bedeutet.

Bevorzugt ist $R_1$ Wasserstoff.

Bevorzugt ist die Verbindung 2-[2-Hydroxy-3,5-di-tert.-octylphenyl]-2H-benzotriazol.

Die Verbindungen der Formel I können wie folgt hergestellt werden:

Stufe I:

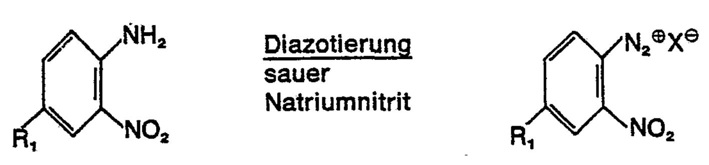


II. III.

III. +

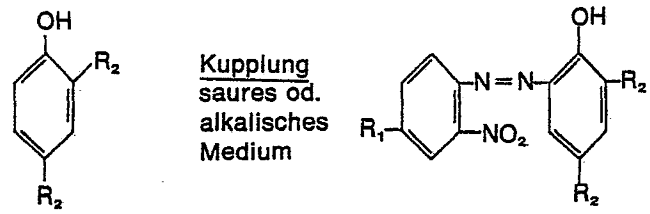


IV. V.

Stufe II:

V. reductive Cyclisierung

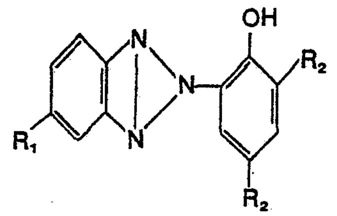

I.

$R_1$ und $R_2$ haben die zuvor genannte Bedeutung.

1. Stufe – Die Kupplung der Diazonium-Verbindung kann in saurem oder alkalischem Milieu erfolgen. Wird die Reaktion in saurem Milieu durchgeführt, so ist die Ausbeute an o-Nitroazobenzol über 70% der Theorie.

2. Stufe – Die reduktive Cyclisierung von V. zu 2-Aryl-2H-benzotriazol kann durch eine der bekannten Reduktionsmethoden, wie z.B. Zink und NaOH, Hydrazin oder katalytische Hydrierung mit einem Edelmetall-Katalysator, erfolgen. Die Verwendung dieser Mittel führt zu guten Benzotriazol-Ausbeuten.

Die Ausgangsprodukte, wie z.B. 2,4-Di-tert.-octylphenol, o-Nitroanilin und 4-Chlor-2-nitroanilin sind im Handel erhältlich oder nach bekannten Methoden auf einfache Weise herzustellen.

Die Verbindungen der Formel I sind wirksame Lichtstabilisatoren für eine grosse Anzahl organischer Polymere. Solche Polymere sind:

1. Polymere, welche von Mono- oder Diolefinen stammen, z.B. gegebenenfalls vernetztes Polyäthylen, Polypropylen, Polyisobutylen, Polymethylbuten-1, Polymethylpenten-1-, Polyisopren und Polybutadien.

2. Gemische der Homopolymere von 1), z.B. Gemische von Polypropylen und Polyethylen, Polypropylen und Polybuten-1, Polypropylen und Polyisobutylen.

3. Copolymere aus den Monomeren für die Homopolymeren unter 1), z.B. Äthylen/Propylen-Copolymer, Propylen/Butene-1-Copolymer, Propylenmer/Isobutylen-Copolymer, Äthylen/Buten-1-Copolymer sowie Terpolymere von Äthylen und Propylen mit einem Dien, z.B. Hexadin, Dicyclopentadien oder Äthyliden-Norbornen, und Copolymere von α-Olefin, z.B. Äthylen mit Acryl- oder Methacrylsäure.

4. Polystyrol.

5. Copolymere von Styrol und von α-Methylstyrol, z.B. Styrol/Butadine-Copolymer, Styrol/Acrylnitril-Copolymer, Styrol/Acrylnitril/Methacrylate-Copolymer, Styrol/Acrylnitril-Copolymer modifiziert mit Acrylester-Polymer, z.B. Styrol/Butadine/Styrol-Block-Copolymer.

6. Graft-copolymere von Styrol, z.B. das Graftpolymer von Styrol zu Polybutadin, das Graft-polymer von Styrol mit Acrylnitril zu Polybutadin sowie ihre Gemische mit den Copolymeren von 5), bekannt als Acrylnitril/Butadin/Styrol oder ABS.

7. Halogenhaltige Vinyl-polymere, z.B. Polyvinylchlorid, Polyvinylidenchlorid, Polyvinylfluorid, Polychloropren, chloriniertes Gummi, Vinylchlorid/Vinylidenchlorid-copolymer, Vinylchlorid/Vinylacetat-copolymer, Vinylidenchlorid/Vinylacetat-copolymer.

8. Polymere, welche von α,β-ungesättigten Säuren stammen und ihre Derivate, Polyacrylate und Polymethacrylate, Polyacrylamide und Polyacrylnitril. Die Verbindungen der Formel I werden vorteilhafterweise in hitzehärtbaren Acrylharz-Lakken verwendet, welche aus einer Acrylsäure und einem Melanin-Formaldehydharz bestehen.

9. Polymere, hergestellt aus ungesättigten Alkoholen und Aminen und von deren Acyl-derivativen oder Acetalen, z.B. Polyvinylalcohol, Polyvinylacetat, Polyvinylstearat, Polyvinylbenzoat, Polyvinylmaleat, Polyvinylbutyral, Polyallylphthalat, Polyallylmelamin und ihre Copolymere mit anderen Vinyl-Verbindungen, z.B. Äthylen/Vinylacetat-copolymer.

10. Homopolymere und Copolymere, hergestellt aus Epoxiden, z.B. Polyäthylenoxid, oder die Polymere der bis-Glycidyläther.

11. Polyacetale, z.B. Polyoxymethylen, sowie Polyoxymethylene, welche Äthylenoxid als Comonomer enthalten.

12. Polyalkylenoxide, z.B. Polyoxyäthylen, Polypropylenoxid oder Polyisobutylenoxid.

13. Polyphenylenoxide.

14. Polyurethane und Polyharnstoffe, wie in Urethanüberzügen.

15. Polycarbonate.

16. Polysulfone.

17. Polyamide und Copolyamide, hergestellt aus Diaminen und Dicarboxylsäuren und/oder aus Aminocarboxylsäuren oder deren Lactamen, z.B.

Polyamid 6, Polyamid 6/6, Polyamid 6/10, Polyamid 11, Polyamid 12, Poly-m-phenylen-isophthalamid.

18. Polyester aus Dicarboxylsäuren und Dialkoholen und/oder aus Hydroxycarboxylsäuren oder deren Lactonen, z.B. Polyäthylenglycolterephthalat, Poly-1,4-dimethylol-cyclohexan-terephthalat.

19. Vernetzte Polymere aus Aldehyden einerseits und aus Phenolen, Harnstoffen und Melamin anderseits, z.B. Phenol/Formaldehyd, Harnstoff/Formaldehyd und Melamin/Formaldehyd-Harz.

20. Alkyd-Harze, z.B. Glycerol/Phthalsäure-Harze und deren Gemische mit Melamin/Formaldehyd-Harzen.

21. Ungesättigte Polyester-Harze aus den Copolyestern der gesättigten und ungesättigten Dicarboxylsäure mit mehrwertigen Alkoholen sowie aus Vinyl-Verbindungen als Vernetzungsmittel und deren halogenhaltigen, flammenresistenten Modifikationen.

22. Natürliche Polymere, z.B. Cellulose, Gummi, sowie deren chemisch modifizierten Homolog-Derivative, z.B. Celluloseacetate, Cellulosepropionate und Cellulosebutyrate und die Celluloseäther, z.B. Methylcellulose.

Die Verbindungen der Formel I sind nicht nur wirksame Lichtschutzmittel, wie die 2-Aryl-2H-benzotriazole im allgemeinen, sondern auch besonders wertvoll zum Stabilisieren von polymeren Substraten, welche bei hohen Temperaturen verarbeitet werden müssen, dank ihres überraschend geringen Verlustes durch Verflüchtigung bei hoher Temperatur.

Die Verbindungen der Formel I sind daher besonders geeignet zum Stabilisieren von Polyestern, wie z.B. Polyäthylenterephthalat, Polybutylenterephthalat oder deren Copolymere, von Polycarbonaten, z.B. aus Bisphenol A und Phosgen oder deren Copolymere, von Polysulfonen, Polyamiden, z.B. Nylon-6, Nylon-6,6, Nylon-6/10 usw. sowie Copolyamide, von duroplastischen Acrylharzen, von thermoplastischen Acrylharzen, von Polyolefinen, z.B. Polyäthylen, Polypropylen, Copolyolefine usw., und von anderen Polymeren, welche Hochtemperatur-Verarbeitung und -Produktion verlangen.

Die Stabilisatoren der Formel I werden nach den herkömmlichen Methoden in das Polymer eingearbeitet, und zwar in jeder beliebigen Phase während der Herstellung von geformten Produkten. Sie können z.B. als Pulver, Suspension oder Emulsion zum Polymer gemischt werden, welches als Pulver, Suspension oder Emulsion vorliegen kann.

Die stabilisierten polymeren Mischungen der Erfindung können gegebenenfalls 0,1 bis 5 Gew.-%, vorzugsweise 0,5 bis 3 Gew.-% der üblichen Zusatzstoffe enthalten, insbesondere Antioxydantien, Lichtstabilisatoren oder deren Gemische. Beispiele für solche Zusatzstoffe:

Antioxydantien, UV-Absorber und Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen, Polyamidstabilisatoren, basische Co-Stabilisatoren, Nukleierungsmittel oder sonstige Zusätze wie z. B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Gewisse hydrophobische, nichtdiffundierende Hydroxyphenylbenzotriazole sind bekannt als UV-Absorber in photographischer Gelatine. (US-PS 3 253 921 und US 4 042 394). Die Verbindungen der Formel I sind durch ihre geringe Flüchtigkeit, durch ihre guten Absorptionseigenschaften im UV-Bereich und durch ihre photographische Inaktivität besonders gut geeignet zum Stabilisieren von Farbbildern.

Von besonderem Interesse sind die duroplastischen und thermoplastischen Acrylharze, welche für Autolackierungen verwendet werden. Diese Stoffe sind in der Encyclopedia of Polymer Science and Technology, Interscience Publishers, New York, Band 1 (1964), auf Seiten 273–276 und Band 13 (1970), auf Seiten 530–532 beschrieben, ferner in «Understanding Paint» von W.R. Fuller, in American Paint Journal Co., St. Louis, 1965, Seite 124–135.

Acrylharzlacke, welche gemäss der Erfindung gegen die Einwirkung von Licht, Sauerstoff und Feuchtigkeit stabilisiert werden, sind die üblichen Einbrennlacke, wie z.B. in H. Kittels «Lehrbuch der Lacke und Beschichtungen», Band 1, Teil 2, Seite 735 und 742 (Berlin, 1972), und in H. Wagner, H.F. Sarx, «Lackkunstharze», Seite 229–235 beschrieben.

Von besonderem Interesse ist die Stabilisierung von Metalleffektlacken auf der Basis von heissvernetzbarem Acrylharz und Styrol mit den Verbindungen der Formel I. Aus diesen Harzen hergestellter, unstabilisierter Metalleffektlack ist trotz der ausgezeichneten physikalischen und chemischen Eigenschaften ungeeignet, da das vorhandene Styrol zur Rissbildung führt. Andere Lacke und heissvernetzbare Beschichtungen sind auf der Basis von Acrylharz, Melaminharz, Alkydharz, Polyesterharz, Epoxyharz oder deren Gemische ebenfalls interessant. Epoxyharz wird nur im Gemisch (maximal 15 Gew.-% Epoxyharz) mit anderen Harzen verwendet.

Um die Metalleffektwirkung zu erzielen, verwendet man die üblichen Aluminium-Pigmente in 1 bis 10 Gew.-%, bezogen auf das lösungsmittelfreie Bindemittel (Lackharz). Das Auftragen der stabilisierten Lacke kann nach den herkömmlichen Einschicht- oder Zweischicht-Verfahren geschehen. Im letzteren Falle wird der Pigment enthaltende Vorlegelack zuerst aufgetragen und nachher mit Klarlack überzogen.

Weitere Zusatzstoffe, die im Lack enthalten sein können, sind andere übliche Lichtschutzmittel, phenolische Antioxydantien, Pigmente, Farbstoffe, Metalldesaktivatoren usw.

Im allgemeinen betragen die erfindungsgemäss verwendeten Stabilisatoren 0,1 bis 5 Gew.-%, vorzugsweise 0,5 bis 3 Gew.-%, bezogen auf das Lackharz.

Die Kombination von sterisch gehinderten Aminen und den Stabilisatoren der Formel I ermöglicht sowohl eine ausgezeichnete Glanzerhaltung als auch eine Beständigkeit gegen Abblätterung der metallisierten Einbrennlacke auf Acrylharzbasis für Automobil-Decklackierungen.

Die sterisch gehinderten Amine sind in den Acrylharzlacken als Lichtstabilisatoren wirksam und verantwortlich für deren Glanzerhaltung bei Bewitterung. Das UV-Licht kann jedoch die Deckschicht, falls kein UV-Absorber vorhanden, ungehindert passieren und in der unteren Epoxyesterschicht zu Schäden führen. Durch die Zugabe der erfindungsgemässen Benzotriazol-UV-Absorbern wird dies erfolgreich verhindert.

Auf diese Weise bietet die Kombination von sterisch gehinderten Aminen mit Benzotriazolen sowohl Glanzerhaltung als auch Abblätterungsbeständigkeit in metallisierten Decklacken auf der Basis von Acrylharzen.

Die eingesetzten Amine sind in 0,1 bis 5 Gew.-%, vorzugsweise in 0,5 bis 2 Gew.-%, insbesondere in 0,5 bis 1 Gew.-% wirksam, bezogen auf das Acrylharz.

Die Benzotriazole der Formel I sind in 0,1 bis 5 Gew.-%, vorzugsweise in 0,5 bis 2 Gew.-%, insbesondere bei 0,5 bis 1 Gew.-% wirksam, bezogen auf das Acrylharz.

Die Amin-Lichtstabilisatoren, die in dieser Erfindung verwendet werden, entsprechen der Formel VI

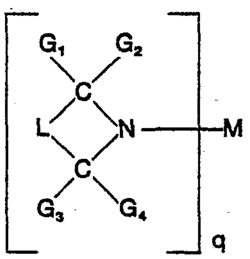 oder 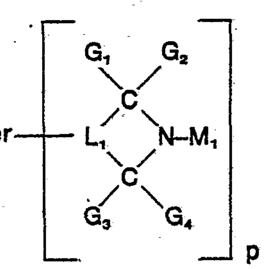

(VI)

worin q 1 oder 2 ist, p 2 bis 14 ist, $G_1$, $G_2$, $G_3$ und $G_4$ unabhängig voneinander Alkyl bedeuten, oder $G_1$ und $G_3$ zusammen ein Alkylen bilden, oder jeder Carboalkoxy oder Carbophenäthoxy ist, oder $G_1$ und $G_2$ oder $G_3$ und $G_4$, unabhängig voneinander, zusammen ein Alkylen oder Azaalkylen bilden; falls q 1 ist, M Wasserstoff, Hydroxy, Oxy, gegebenenfalls substituiertes Alkyl, Alkenyl, Alkinyl, Aralkyl, Alkanoyl, Alkenoyl, Benzoyl, Glycidyl oder $-CH_2CHOHZ$ ist, wo Z Wasserstoff, Methyl oder Phenyl bedeutet, falls q 2 ist, M Alkylen, Alkenylen, Alkinylen, Arylendialkylen, die Gruppe $-(CH_2)_2OOCR_{18}COO(CH_2)_2-$, oder die Gruppe $-CH_2OOOCR_{19}COOCH_2-$ ist, worin $R_{18}$ Alkylen und $R_{19}$ Alkylen, Xylylen oder Cyclohexylen sind, $M_1$ die gleiche Bedeutung wie M hat, wo q 1 ist,

L ein zweiwertiger organischer Rest ist, welcher den N-haltigen Ring zu einem 5- bis 7-Ring ergänzt, oder zwei einwertige Radikale bedeutet, und

$L_1$ ein zweiwertiger organischer Rest, welcher den N-haltigen Ring zu einem 5- bis 7-Ring ergänzt und welcher zusätzlich durch eine Verbindungsgruppe mit anderen Amingruppierungen verbunden sein kann.

Als Amine kommen die Verbindungen der folgenden Formel in Frage:

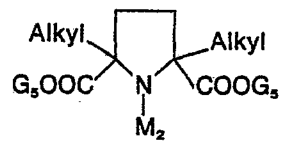 (VII),

worin $G_5$ Wasserstoff, Alkyl oder Phenäthyl ist und $M_2$ Wasserstoff, Hydroxy, Oxy, Alkyl, 2-Methoxyäthyl, Alkenyl oder Propargyl bedeutet.

Bevorzugt werden 2,2,6,6-Tetraalkylpiperidinverbindungen verwendet, welche vorzugsweise eine Gruppe der Formel VIII

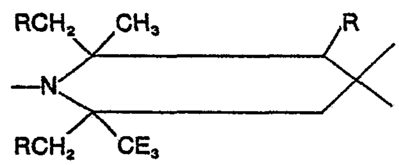 (VIII)

enthalten, worin R Wasserstoff oder Methyl bedeutet.

Zu den erfindungsgemäss zu verwendenden Lichtschutzmitteln gehören insbesondere folgende Verbindungsklassen:

a) Lichtschutzmittel der Formel IX

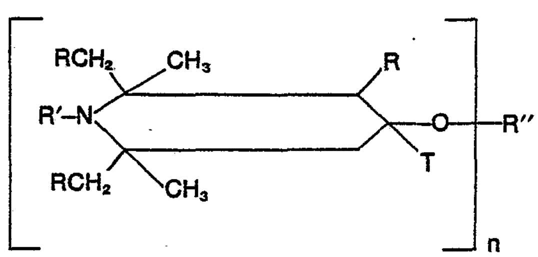 (IX)

worin n die Zahlen 1 bis 4, vorzugsweise 1 oder 2 bedeutet, T Wasserstoff oder $-CONH_2$ ist, R die für Formel VIII angegebene Bedeutung hat, R' Wasserstoff, Oxy, $C_1$–$C_{18}$ Alkyl, $C_3$–$C_8$ Alkenyl, $C_3$–$C_8$ Alkinyl, $C_7$–$C_{12}$ Aralkyl, $C_1$–$C_8$ Alkanoyl, $C_3$–$C_5$ Alkenoyl, Glycidyl, eine Gruppe $-CH_2CH(OH)-Z$, worin Z Wasserstoff, Methyl, Äthyl oder Phenyl ist, wobei R' vorzugsweise Wasserstoff, $C_1$–$C_{12}$ Alkyl, Allyl, Benzyl, Acetyl, Acryloyl ist und R'', wenn n = 1, Wasserstoff, gegebenenfalls durch ein oder mehrere Sauerstoffatome unterbrochenes $C_1$–$C_{18}$ Alkyl, Cyanäthyl, Benzyl, Glycidyl, einen einwertigen Rest einer aliphatischen, cycloaliphatischen, araliphatischen, aromatischen oder heterocyclischen Carbonsäure, Carbaminsäure oder Phosphor enthaltenden Säure oder einen einwertigen Silylrest, vorzugsweise

einen Rest einer aliphatischen Carbonsäure mit 2 bis 18 C-Atomen, einer cycloaliphatischen oder aromatischen Dicarbon- oder einer aromatischen Carbonsäure mit 6 bis 15 C-Atomen, wenn n = 2, $C_1$–$C_{12}$ Alkylen, $C_4$–$C_{12}$ Alkenylen, Xylylen, einen zweiwertigen Rest einer aliphatischen, cycloaliphatischen, araliphatischen, aromatischen oder heterocyclischen Dicarbonsäure, Dicarbaminsäure oder Phosphor enthaltenden Säure oder einen zweiwertigen Silylrest, vorzugsweise einen Rest einer aliphatischen Dicarbonsäure mit 2 bis 36 C-Atomen, einer cycloaliphatischen oder aromatischen Dicarbonsäure mit 8 bis 10 C-Atomen, einer aliphatischen, cycloaliphatischen oder aromatischen Dicarbaminsäure mit 8 bis 10 C-Atomen, wenn n = 3, einen dreiwertigen Rest einer aliphatischen, cycloaliphatischen, aromatischen oder heterocyclischen Tricarbonsäure, einer aromatischen Tricarbaminsäure oder einer Phosphor enthaltenden Säure oder einen dreiwertigen Silylrest, wenn n = 4, einen vierwertigen Rest einer aliphatischen, cycloaliphatischen oder aromatischen Tetracarbonsäure bedeutet.

Bedeuten etwaige Substituenten $C_1$–$C_{12}$ Alkyl, so stellen sie z.B. Methyl, Äthyl, n-Propyl, n-Butyl, sek.-Butyl, tert.-Butyl, n-Hexyl, n-Octyl, 2-Äthylhexyl, n-Nonyl, n-Decyl, n-Undecyl oder n-Dodecyl dar.

In der Bedeutung von $C_1$–$C_{18}$ Alkyl können R′ oder R″ z.B. die oben angeführten Gruppen und dazu noch beispielsweise n-Tridecyl, n-Tetradecyl, n-Hexadecyl oder n-Octadecyl darstellen.

Wenn R′ $C_3$–$C_8$ Alkenyl bedeutet, so kann es sich z.B. um 1-Propenyl, Allyl, Methallyl, 2-Butenyl, 2-Pentenyl, 2-Hexenyl, 2-Octenyl, 4-tert.-Butyl-2-butenyl handeln.

R′ ist als $C_3$–$C_8$ Alkinyl bevorzugt Propargyl.

Als $C_7$–$C_{12}$ Aralkyl ist R′ insbesondere Phenäthyl oder vor allem Benzyl.

R′ ist als $C_1$–$C_8$ Alkanoyl beispielsweise Formyl, Propionyl, Butyryl, Octanoyl, aber bevorzugt Acetyl und als $C_3$–$C_5$ Alkenoyl, insbesondere Acryloyl.

Bedeutet R″ einen einwertigen Rest einer Carbonsäure, so stellt es beispielsweise einen Essigsäure-, Stearinsäure-, Salicylsäure-, Methacrylsäure-, Maleinsäure-, Benzoe- oder β-(3,5-Di-tert.-butyl-4-hydroxyphenyl)-propionsäurerest dar.

Bedeutet R″ einen zweiwertigen Rest einer Dicarbonsäure, so stellt es beispielsweise einen Adipinsäure-, Suberinsäure-, Sebacinsäure-, Phthalsäure-, Dibutylmalonsäure-, Dibenzylmalonsäure-, Butyl-(3,5-di-tert.-butyl-4-hydroxybenzyl)-malonsäure- oder Bicycloheptendicarbonsäurerest dar.

Stellt R″ einen dreiwertigen Rest einer Tricarbonsäure dar, so bedeutet es z.B. einen Trimellithsäure- oder einen Nitrilotriessigsäurerest.

R″ kann auch Reste von Di- und Tricarbonsäure bedeuten, wie sie technisch durch Oligomerisation höherer ungesättigter Fettsäuren oder durch Diels-Alder Addition von Acrylsäure an Linolsäure hergestellt werden. Ferner kann R″ auch Reste bedeuten, die durch Umsetzung von Mono-, Di- und Polyepoxiden wie z.B. Bisphenol-A-diglycidyläther, Butandialdiglycidyläther, Tris-glycidyl-isocyanurat, 1,3-Diglycidyl-4,4-dimethyl-hydantoin mit 4-Hydroxy-1,2,2,6,6-pentamethylpiperidin entstehen.

Stellt R″ einen vierwertigen Rest einer Tetracarbonsäure dar, so bedeutet es z.B. einen Pyromellithsäurerest.

Bedeutet R″ einen zweiwertigen Rest einer Dicarbaminsäure, so stellt es beispielsweise einen Hexamethylendicarbaminsäure- oder einen 2,4-Toluylen-dicarbaminsäurerest dar.

Beispiele für Polyalkylpiperidin-Lichtschutzmittel dieser Klasse sind folgende Verbindungen:

1) 4-Hydroxy-2,2,6,6-tetramethylpiperidin
2) 1-Allyl-4-hydroxy-2,2,6,6-tetramethylpiperidin
3) 1-Benzyl-4-hydroxy-2,2,6,6-tetramethylpiperidin
4) 1-(4-tert.-Butyl-2-butenyl)-4-hydroxy-2,2,6,6-tetramethylpiperidin
5) 4-Stearoyloxy-2,2,6,6-tetramethylpiperidin
6) 1-Äthyl-4-salicyloyloxy-2,2,6,6-tetramethylpiperidin
7) 4-Methacryloyloxy-1,2,2,6,6-pentamethylpiperidin
8) 1,2,2,6,6-Pentamethylpiperidin-4-yl-β-(3,5-di-tert.-butyl-4-hydroxyphenyl)-propionat
9) 1-Benzyl-2,2,6,6-tetramethyl-4-piperidinylmaleinat
10) Bis-(2,2,6,6-tetramethylpiperidin-4-yl)-adipat
11) Bis-(2,2,6,6-tetramethylpiperidin-4-yl)-sebacat
12 Bis-(1,2,3,6-tetramethyl-2,6-diäthyl-piperidin-4-yl)-sebacat
13) Bis-(1-allyl-2,2,6,6-tetramethyl-piperidin-4-yl)-phthalat
14) 1-Propargyl-4-β-cyanoäthyloxy-2,2,6,6-tetramethylpiperidin
15) 1-Acetyl-2,2,6,6-tetramethylpiperidin-4-yl-acetat
16) Trimellithsäure-tri-(2,2,6,6-tetramethylpiperidin-4-yl)-ester
17) 1-Acryloyl-4-benzyloxy-2,2,6,6-tetramethylpiperidin
18) Dibutyl-malonsäure-di-(1,2,2,6,6-pentamethyl-piperidin-4-yl)-ester
19) Butyl-(3,5-di-tert.-butyl-4-hydroxybenzyl)-malonsäure-di-(1,2,2,6,6-pentamethylpiperidin-4-yl)-ester
20) Dibenzyl-malonsäure-di-(1,2,2,6,6-pentamethylpiperidin-4-yl)-ester
21) Dibenzyl-malonsäure-di-(1,2,3,6-tetramethyl-2,6-diäthyl-piperidin-4-yl)-ester
22) Hexan-1′,6′-bis-(4-carbamoyloxy-1-n-butyl-2,2,6,6-tetramethylpiperidin)
23) Toluol-2′,4′-bis-(4-carbamoyloxy-1-n-propyl-2,2,6,6-tetramethylpiperidin)
24) Dimethyl-bis-(2,2,6,6-tetramethylpiperidin-4-oxy)-silan
25) Phenyl-tris-(2,2,6,6-tetramethylpiperidin-4-oxy)-silan
26) Tris-(1-propyl-2,2,6,6-tetramethylpiperidin-4-yl)-phosphit
27) Tris-(1-propyl-2,2,6,6-tetramethylpiperidin-4-yl)-phosphat

28) Phenyl-[bis-(1,2,2,6,6-pentamethylpiperidin-4-yl)]-phosphonat
29) Bis-(1,2,2,6,6-pentamethylpiperidin-4-yl)-sebacat
30) Bis-(1-benzyl-2,2,6,6-tetramethylpiperidin-4-yl)-sebacat
31) Bis-(1-allyl-2,2,6,6-tetramethylpiperidin-4-yl)-sebacat
31a) 2,2,6,6-Tetramethyl-4-hydroxy-4-carbamoyl-piperidin
32) 4-Benzoyloxy-2,2,6,6-tetramethylpiperidin
33) 4-Acryloxy-2,2,6,6-tetramethylpiperidin
34) 4-(p-Chlorbenzoyloxy)-1,2,2,6,6-pentamethylpiperidin
35) 4-Lauroyloxy-1,2,2,6,6-pentamethylpiperidin
36) 1-Allyl-2,2,6,6-tetramethyl-4-piperidinyl-salicylat
37) 1-(2-Benzoyloxyäthyl)-2,2,6,6-tetramethyl-4-piperidinylbenzoat
38) Bis-(1,2,2,6,6-pentamethyl-4-piperidinyl)-isophthalat
39) Bis-(1,2,2,6,6-pentamethyl-4-piperidinyl)-adipat
40) Bis-(1-acetyl-2,2,6,6-tetramethyl-4-piperidinyl)-sebacat
41) Bis-(1-hydroxyäthyl-2,2,6,6-tetramethyl-4-piperidinyl)-succinat
42) Tris-(2,2,6,6-tetramethyl-4-piperidinyl)-nitrilotriacetat
43) Tris-(1-butyl-2,2,6,6-tetramethyl-4-piperidinyl)-trimellithat
44) Tris-(1-butyl-2,2,6,6-tetramethyl-4-piperidinyl)-phosphat
45) Diphenyl-bis-(2,2,6,6-tetramethylpiperidin-4-oxy)-silanester
46) O,O′-Di-(2,2,6,6-tetramethyl-4-piperidinyl)-tolylen-1,4-dicarbamat
47) 1-Methylcarbamoyl-2,3,6-trimethyl-2,6-diäthyl-4-piperidinylmethylcarbamat.

b) Lichtschutzmittel der Formel X

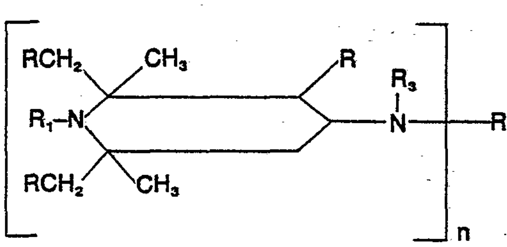

(X)

worin n die Zahlen 1 oder 2 bedeutet, R die für Formel VIII angegebene Bedeutung hat, R′ die unter a) angegebene Bedeutung hat, $R_3$ Wasserstoff, $C_1$–$C_{12}$ Alkyl, $C_5$–$C_7$ Cycloalkyl, $C_7$–$C_8$ Aralkyl, $C_2$–$C_{18}$ Alkanoyl, $C_3$–$C_5$ Alkenoyl, Benzoyl ist und $R_4$, wenn n = 1, Wasserstoff, $C_1$–$C_{18}$ Alkyl, $C_5$–$C_7$ Cycloalkyl, gegebenenfalls mit einer Cyano-, Carbonyl oder Carbamidgruppe substituiertes $C_2$–$C_8$ Alkenyl, Glycidyl, eine Gruppe der Formel $-CH_2-CH(OH)-Z$ oder der Formel $-CONH-Z$ ist, worin Z Wasserstoff, Methyl oder Phenyl bedeutet; wenn n = 2, $C_2$–$C_{12}$ Alkylen, $C_6$–$C_{12}$ Arylen, Xylylen, eine $-CH_2-CH(OH)-CH_2$-Gruppe oder eine Gruppe $-CH_2-CH(OH)-CH_2-O-X-O-CH_2-CH(OH)-CH_2-$ bedeutet, worin X $C_2$–$C_{10}$ Alkylen, $C_6$–$C_{15}$ Arylen, $C_6$–$C_{12}$ Cycloalkylen ist, oder, vorausgesetzt, dass $R_3$ nicht Alkanoyl, Alkenoyl oder Benzoyl bedeutet, $R_4$ auch einen zweiwertigen Rest einer aliphatischen, cycloaliphatischen oder aromatischen Dicarbonsäure oder Dicarbaminsäure oder auch die Gruppe –CO– bedeuten kann, oder $R_3$ und $R_4$ zusammen, wenn n = 1, den Imidrest einer aliphatischen, cycloaliphatischen oder aromatischen 1,2- oder 1,3-Dicarbonsäure sein können.

Stellen etwaige Substituenten $C_1$–$C_{18}$ Alkyl dar, so haben sie die bereits unter a) angegebene Bedeutung.

Bedeuten etwaige Substituenten $C_5$–$C_7$ Cycloalkyl, so stellen sie insbesondere Cyclohexyl dar. Als $C_7$–$C_8$ Aralkyl ist $R_3$ insbesondere Phenäthyl oder vor allem Benzyl.

$R_3$ ist als $C_2$–$C_{18}$ Alkanoyl beispielsweise Propionyl, Butyryl, Octanoyl, Dodecanoyl, Hexadecanoyl, Octadecanoyl, aber bevorzugt Acetyl und als $C_3$–$C_5$ Alkenoyl insbesondere Acryloyl.

Bedeutet $R_4$ ein gegebenenfalls mit einer Cyano-, Carbonyl- oder Carbamidgruppe substituiertes $C_2$–$C_8$ Alkenyl, dann handelt es sich z.B. um 1-Propenyl, Allyl, Methallyl, 2-Butenyl, 2-Pentenyl, 2-Hexenyl, 2-Octenyl, 2,2-Dicyanovinyl, 1-Methyl-2-cyano-2-methoxycarbonyl-vinyl, 2,2-Diacetylaminovinyl.

Stellen etwaige Substituenten $C_2$–$C_{12}$ Alkylen dar, so handelt es sich z.B. um Äthylen, Propylen, 2,2-Dimethylpropylen, Tetramethylen, Hexamethylen, Octamethylen, Decamethylen oder Dodecamethylen.

Bedeuten etwaige Substituenten $C_6$–$C_{15}$ Arylen, so stellen sie z.B. o-, m- oder p-Phenylen, 1,4-Naphthylen oder 4,4′-Diphenylen dar.

Als $C_6$–$C_{12}$ Cycloalkylen ist X insbesondere Cyclohexylen.

Beispiele für Polyalkylpiperidin-Lichtschutzmittel dieser Klasse sind folgende Verbindungen:

48) N,N′-Bis-(2,2,6,6-tetramethylpiperidin-4-yl)-hexamethylen-1,6-diamin
49) N,N′-Bis-(2,2,6,6-tetramethylpiperidin-4-yl)-hexamethylen-1,6-diacetamid
50) 4-Acetylamino-1,2,2,6,6-pentamethylpiperidin
51) 1-Benzyl-2,2,6,6-tetramethyl-4-diäthanolamino-piperidin
52) 4-Acrylamido-1,2,2,6,6-pentamethylpiperidin
53) 1-Acetyl-4-(N-cyclohexylacetamido)-2,2,6,6-tetramethylpiperidin
54) 4-Benzylamino-2,2,6,6-tetramethylpiperidin
55) N,N′-Bis-(2,2,6,6-tetramethylpiperidin-4-yl)-N,N′-dibutyl-adipamid
56) N,N′-Bis-(2,2,6,6-tetramethylpiperidin-4-yl)-N,N′-dicyclohexyl-(2-hydroxypropylen)
57) N,N′-Bis-(2,2,6,6-tetramethylpiperidin-4-yl)-p-xylylen-diamin

58) N,N'-Bis-(1,2,2,6,6-pentamethyl-4-piperidinyl)-1,6-diaminohexan.

Die Verbindung der Formel XI

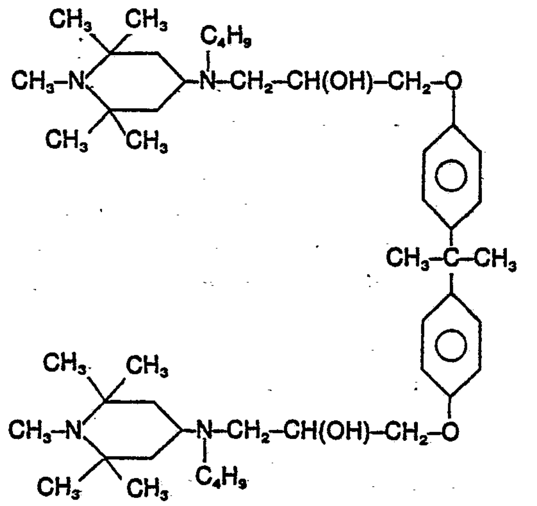

(XI)

59) 4-(Bis-2-hydroxyäthyl)-amino-1,2,2,6,6-pentamethylpiperidin

60) 4-(3-Methyl-4-hydroxy-5-tert.-butylbenzoesäure-amido)-2,2,6,6-tetramethyl-piperidin

61) 4-Methacrylamido-1,2,2,6,6-pentamethyl-piperidin

62) α-Cyano-β-methyl-β-[N-(2,2,6,6-tetra-methylpiperidin-4-yl)]-amino-acrylsäure-methylester

c) Lichtschutzmittel der Formel XII

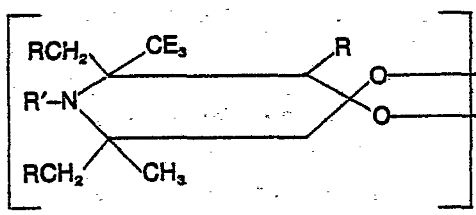

(XII)

worin n die Zahlen 1 oder 2 bedeutet, R die für Formel VIII angegebene Bedeutung hat, R' die unter a) angegebene Bedeutung hat und $R_5$, wenn n = 1, $C_2$–$C_8$ Alkylen oder Hydroxyalkylen, $C_4$–$C_{22}$ Acyloxyalkylen, wenn n = 2, die Gruppe $(-CH_2)_2C(CH_2-)_2$ bedeutet.

Bedeutet $R_5$ $C_2$–$C_8$ Alkylen oder Hydroxyalkylen, so stellt es beispielsweise Äthylen, 1-Methyl-äthylen, Propylen, 2-Äthylpropylen, 2-Äthyl-2-hydroxymethylpropylen dar.

Als $C_4$–$C_{22}$ Acyloxyalkylen bedeutet $R_5$ z.B. 2-Äthyl-2-acetoxymethyl-propylen.

Beispiele für Polyalkylpiperidin-Lichtschutzmittel dieser Klasse sind folgende Verbindungen:

63) 9-Aza-8,8,10,10-tetramethyl-1,5-dioxa-spiro[5.5]undecan

64) 9-Aza-8,8,10,10-tetramethyl-3-äthyl-1,5-dioxaspiro[5.5]decan

65) 8-Aza-2,7,7,8,9,9-hexamethyl-1,4-dioxy-spiro[4.5]decan

66) 9-Aza-3-hydroxymethyl-3-äthyl-8,8,9,10,10-pentamethyl-1,5-dioxaspiro[5.5]undecan

67) 9-Aza-3-äthyl-3-acetoxymethyl-9-acetyl-8,8,10,10,-tetramethyl-1,5-dioxaspiro[5.5]undecan

68) 2,2,6,6-Tetramethylpiperidin-4-spiro-2'-(1',3'-dioxan)-5'-spiro-5''-(1'',3''-dioxan)-2''-spiro-4'''-(2''',2''',6''',6'''-tetra-methyl-piperidin).

d) Lichtschutzmittel der Formeln XIII A, XIII B und XIII C

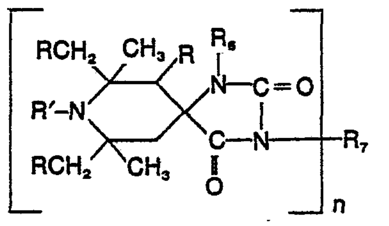

(XIII A)

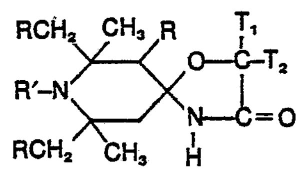

(XIII B)

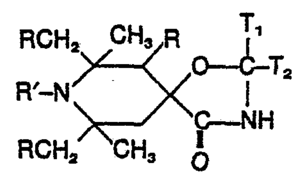

(XIII C)

worin n die Zahlen 1 oder 2 bedeutet, R die für Formel VIII angegebene Bedeutung hat, R' die unter a) angegebene Bedeutung hat, $R_6$ Wasserstoff, $C_1$–$C_{12}$ Alkyl, Allyl, Benzyl, Glycidyl, $C_2$–$C_6$ Alkoxyalkyl ist und $R_7$, wenn n = 1, Wasserstoff, $C_1$–$C_{12}$ Alkyl, $C_3$–$C_5$ Alkenyl, $C_7$–$C_9$ Aralkyl, $C_5$–$C_7$ Cycloalkyl, $C_2$–$C_4$ Hydroxyalkyl, $C_2$–$C_6$ Alkoxyalkyl, $C_6$–$C_{10}$ Aryl, Glycidyl, eine Gruppe der Formel $-(CH_2)_m-COO-Q$ oder der Formel $-(CH_2)_m-O-CO-Q$, worin m 1 oder 2 und Q $C_1$–$C_{18}$ Alkyl oder Phenyl sind, wenn n = 2, $C_2$–$C_{12}$ Alkylen, $C_6$–$C_{12}$ Arylen, $C_4$–$C_{12}$ Alkenylen, eine Gruppe

$-CH_2-CH(OH)-CH_2-O-X-O-CH_2-CH(OH)-CH_2-$

worin X $C_2$–$C_{10}$ Alkylen, $C_6$–$C_{15}$ Arylen, $C_6$–$C_{12}$ Cycloalkylen ist, oder eine Gruppe

$-CH_2CH(OZ')CH_2-(OCH_2-CH(OZ')CH_2)_2-$

bedeutet, worin Z' Wasserstoff, $C_1$–$C_{18}$ Alkyl, Allyl, Benzyl, $C_2$–$C_{12}$ Alkanoyl oder Benzoyl ist, $T_1$ und $T_2$ unabhängig voneinander Wasserstoff, $C_1$–$C_{18}$ Alkyl oder gegebenenfalls durch Halogen oder $C_1$–$C_4$ Alkyl substituiertes $C_6$–$C_{10}$ Aryl oder $C_7$–$C_9$ Aralkyl bedeuten oder $T_1$ und $T_2$ zusammen mit dem sie bindenden C-Atom gegebenenfalls durch $C_1$–$C_4$ Alkyl substituiertes $C_5$–$C_7$ Cycloalkyl, Pyrrolidinyl oder Piperidinyl bilden.

Bedeuten etwaige Substituenten $C_1$–$C_{12}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl oder n-Dodecyl dar.

Etwaige Substituenten in der Bedeutung von $C_1$–$C_{18}$ Alkyl können z.B. die oben angeführten Gruppen und dazu noch beispielsweise n-Tridecyl, n-Tetradecyl, n-Hexadecyl oder n-Octadecyl darstellen.

Bedeuten etwaige Substituenten $C_2$–$C_6$ Alkoxyalkyl, so stellen sie z.B. Methoxymethyl, Äthoxymethyl, Propoxymethyl, tert.-Butoxymethyl, Äthoxyäthyl, Äthoxypropyl, n-Butoxyäthyl, tert.-Butoxyäthyl, Isopropoxyäthyl oder Propoxypropyl dar.

Stellt $R_7$ $C_3$–$C_5$ Alkenyl dar, so bedeutet es z.B. 1-Propenyl, Allyl, Methallyl, 2-Butenyl oder 2-Pentenyl.

Als $C_7$–$C_9$ Aralkyl sind $R_7$, $T_1$ und $T_2$, insbesondere Phenäthyl oder vor allem Benzyl und als $C_5$–$C_7$ Cycloalkyl ($T_1$ und $T_2$ zusammen mit dem sie bindenden C-Atom), insbesondere Cyclohexyl.

Bedeutet $R_7$ $C_2$–$C_4$ Hydroxyalkyl, so stellt es z.B. 2-Hydroxyäthyl, 2-Hydroxypropyl, 2-Hydroxypropyl, 2-Hydroxybutyl oder 4-Hydroxybutyl dar.

Als $C_6$–$C_{10}$ Aryl bedeuten $R_7$, $T_1$ und $T_2$ insbesondere Phenyl, α- oder β-Naphthyl, die gegebenenfalls mit Halogen oder $C_1$–$C_4$ Alkyl substituiert sind.

Stellt $R_7$ $C_2$–$C_{12}$ Alkylen dar, so handelt es sich z.B. um Äthylen, Propylen, 2,2-Dimethylpropylen, Tetramethylen, Hexamethylen, Octamethylen, Decamethylen oder Dodecamethylen.

Als $C_4$–$C_{12}$ Alkenylen bedeutet $R_7$ insbesondere 2-Butenylen, 2-Pentenylen oder 3-Hexenylen.

Bedeutet $R_7$ $C_6$–$C_{12}$ Arylen, so stellt es beispielsweise o-, m- oder p-Phenylen, 1,4-Naphthylen oder 4,4'-Diphenylen dar.

Bedeutet Z' $C_2$–$C_{12}$ Alkanoyl, so stellt es beispielsweise Propionyl, Butyryl, Octanoyl, Dodecanoyl, aber bevorzugt Acetyl dar.

X hat als $C_2$–$C_{10}$ Alkylen, $C_6$–$C_{15}$ Arylen oder $C_6$–$C_{12}$ Cycloalkylen die unter b) angegebene Bedeutung.

Beispiele für Polyalkylpiperidin-Lichtschutzmittel dieser Klasse sind folgende Verbindungen:

69) 3-Benzyl-1,3,8-Triaza-7,7,9,9-tetramethyl-spiro[4,5]decan-2,4-dion
70) 3-n-Octyl-1,3,8-triaza-7,7,9,9-tetramethyl-spiro[4.5]decan-2,4-dion
71) 3-Allyl-1,3,8-triaza-1,7,7,9,9-pentamethyl-spiro[4.5]decan-2,4-dion
72) 3-Glycidyl-1,3,8-triaza-7,7,8,9,9-pentamethylspiro[4.5]decan-2,4-dion
73) 2-iso-Propyl-7,7,9,9-tetramethyl-1-oxa-3,8-diaza-4-oxo-spiro[4,5]decan
74) 2-Butyl-7,7,9,9-tetramethyl-1-oxa-3,8-diaza-4-oxo-spiro[4,5]decan
75) 2-Isopropyl-7,7,9,9-tetramethyl-1-oxa-4,8-diaza-3-oxo-spiro[4,5]decan
76) 2-Butyl-7,7,9,9-tetramethyl-1-oxa-4,8-diaza-3-oxo-spiro[4,5]decan,

sowie die folgenden Verbindungen:

77) 2,2,4,4-Tetramethyl-7-oxa-3,20-diaza-21-oxo-dispiro-[5,1,11,−2]-heneicosan
78) 1,3-Di-(2,2,6,6-tetramethyl-4-piperidinyl)-imidazolidinon-2
79 2,4,6-Tri-[N-(2,2,6,6-tetramethyl-4-piperidinyl)-butylamino]-s-triazin
80) Di-[2-(2,2,6,6-tetramethyl-1-piperidino)-äthyl]-adipat
81) 2,2,6,6-Tetramethylpiperidin-1-essigsäure-n-octylester.

Weitere Verbindungen der Formel:

82) 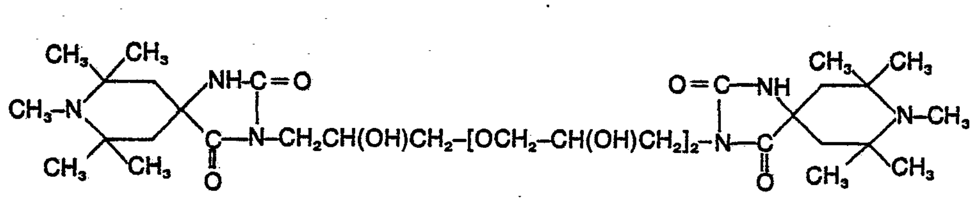

83) 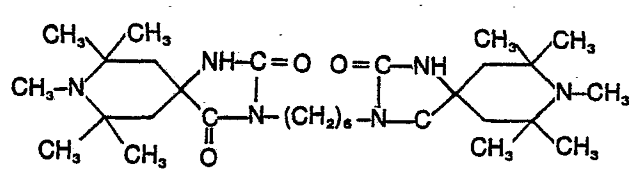

84) 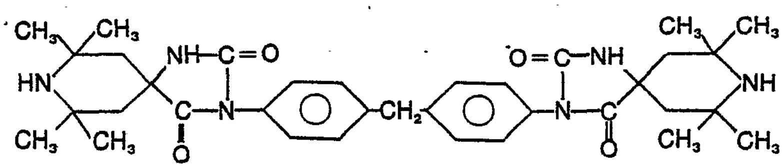

e) Lichtschutzmittel der Formel XIV

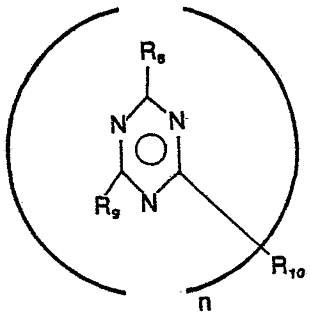

(XIV)

worin n die Zahlen 1 oder 2 ist und $R_8$ eine Gruppe der Formel

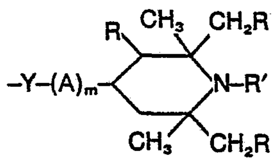

bedeutet, worin R die für Formel (I) angegebene Bedeutung hat, R′ die unter a) angegebene Bedeutung hat, Y –O– oder $-NR_{11}-$ ist, A $C_2$–$C_6$ Alkylen oder $-(CH_2)_3-O-$ und m die Zahlen 0 oder 1 bedeuten, $R_9$ die Gruppen $R_8$, $NR_{11}R_{12}$, $-OR_{13}$, $-NHCH_2OR_{13}$ oder $-N(CH_2OR_{13})_2$ ist, $R_{10}$, wenn n = 1 die Gruppen $R_8$ oder $R_9$ und wenn n = 1 die Gruppen $R_8$ oder $R_9$ und wenn n = 2 die Gruppe –Y–Q–Y–, worin Q gegebenenfalls durch $-N(R_{14})-$ unterbrochenes $C_2$–$C_6$ Alkylen bedeutet, $R_{11}$ $C_1$–$C_{12}$ Alkyl, Cyclohexyl, Benzyl oder $C_1$–$C_4$ Hydroxyalkyl oder eine Gruppe der Formel

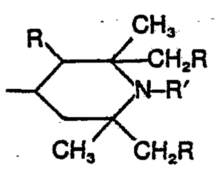

ist, $R_{12}$ $C_1$–$C_{12}$ Alkyl, Cyclohexyl, Benzyl, $C_1$–$C_4$ Hydroxyalkyl, $R_{13}$ Wasserstoff, $C_1$–$C_{12}$ Alkyl oder Phenyl und $R_{14}$ Wasserstoff, $C_1$–$C_{12}$ Alkyl, Cyclohexyl, Benzyl, Phenyl bedeuten oder $R_{11}$ und $R_{12}$ zusammen $C_4$–$C_5$ Alkylen oder Oxaalkylen sind oder auch $R_{11}$ und $R_{12}$ jeweils eine Gruppe der Formel

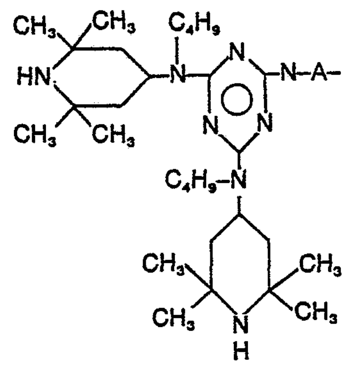

bedeuten.

Bedeuten etwaige Substituenten $C_1$–$C_{12}$ Alkyl, so stellen sie beispielsweise Methyl, Äthyl, n-Propyl, n-Butyl, sek.-Butyl, tert.-Butyl, n-Hexyl, n-Octyl, 2-Äthylhexyl, n-Nonyl, n-Decyl, n-Undecyl oder n-Dodecyl dar.

Bedeuten etwaige Substituenten $C_1$–$C_4$ Hydroxyalkyl, so stellen sie z.B. 2-Hydroxyäthyl, 2-Hydroxypropyl, 3-Hydroxypropyl, 2-Hydroxybutyl oder 4-Hydroxybutyl dar.

Bedeutet A $C_2$–$C_6$ Alkylen, so stellt es beispielsweise Äthylen, Propylen, 2,2-Dimethylpropylen, Tetramethylen oder Hexamethylen dar.

Stellen $R_{11}$ und $R_{12}$ zusammen $C_4$–$C_5$ Alkylen oder Oxaalkylen dar, so bedeutet dies z.B. Tetramethylen, Pentamethylen oder 3-Oxa-pentamethylen.

Verbindungen der Formeln XV oder XVI

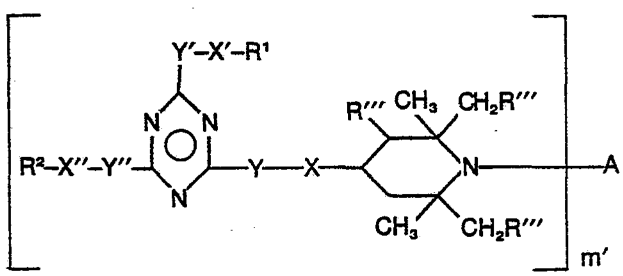

(XV)

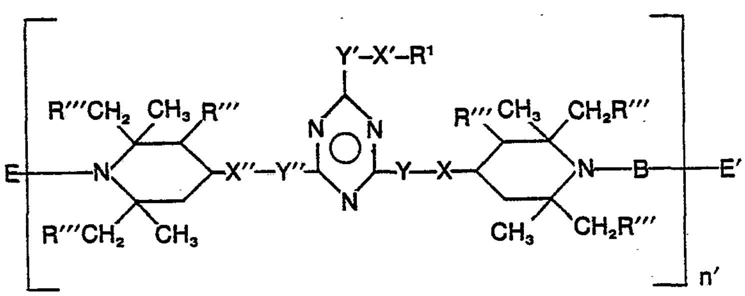

(XVI)

worin

m' 2, 3 oder 4 ist, n' 2 bis 50 ist,

X,X' und X'' eine direkte Bindung, $C_1$–$C_4$ Alkylen oder $-OCH_2CH_2CH_2-$, dessen O nicht an Y, Y' oder Y'' gebunden ist, darstellen

Y, Y' und Y'' –O–, –S–, $-\underset{|}{N}H-$ oder $-NR^3-$ darstellen

R''' Wasserstoff oder $C_1$–$C_4$ Alkyl bedeutet,

$R^1$, $R^2$ und $R^3$ $C_1$–$C_{12}$ Alkyl, $C_2$–$C_8$ Alkoxyalkyl, $C_2$–$C_4$ Hydroxyalkyl, $C_5$–$C_{10}$ Cycloalkyl, $C_6$–$C_{10}$ Aryl, durch 1 oder 2 $C_1$–$C_8$ Alkylgruppen und/oder OH und/oder $C_1$–$C_4$ Alkoxy substituiertes Phenyl oder eine Polyalkylpiperidinylgruppe der Formel

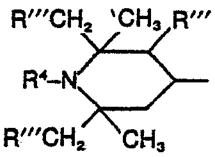

darstellt

oder im Falle, dass Y' oder Y'' $-NR^3-$ ist und X' oder X'' eine direkte Bindung ist, $R^1$ und $R^3$ zusammen mit dem N-Atom einen Pyrrolidin-, Piperidin- oder Morpholinring bilden,

$R^4$ Wasserstoff, $O^{\cdot}$, $C_1$–$C_{12}$ Alkyl, Allyl oder Benzyl bedeutet,

A, wenn m 2 ist, $C_2$–$C_{12}$ Alkylen, $C_4$–$C_8$ Alkenylen, Xylylen oder einen Rest der Formel

$-CH_2-COO-R^5-OOC-CH_2-$, $-CH_2-CH(OH)-CH_2-$ oder

$-CH_2CH(OH)CH_2-D-CH_2CH(OH)CH_2-$

darstellt, wenn m 3 ist, eine Gruppe der Formel

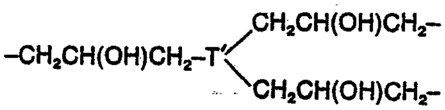

darstellt und

wenn m 4 ist, eine Gruppe der Formel

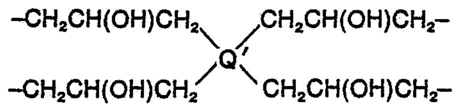

darstellt,

B $C_2$–$C_{12}$ Alkylen, $C_4$–$C_8$ Alkenylen, Xylylen oder einen Rest der Formel

$-CH_2-COO-R^5-OOC-CH_2-$,

$-CH_2-CH(OH)-CH_2-$ oder

$-CH_2CH(OH)CH_2-D-CH_2CH(OH)CH_2-$

darstellt,

$R^5$ $C_2$–$C_8$ Alkylen, $C_4$–$C_8$ Oxaalkylen oder Cyclohexylen darstellt,

D einen zweiwertigen Rest der Formel

$-O-R^6-O-$, $-O-C(O)-R^7-C(O)-O-$,

$-OCH(R^8)CH_2O-R^6-OCH_2CH(R^8)O-$ oder

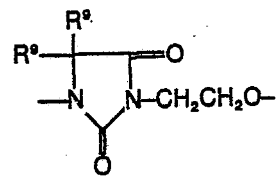

darstellt,

$R^6$ $C_2$–$C_{12}$ Alkylen, $C_6$–$C_{12}$ Cycloalkylen, $C_6$–$C_{12}$ Arylen oder –Phenylen–Z'–Phenylen– darstellt, worin Z' $-CH_2-$, $C(CH_3)_2$, $-SO_2-$ oder –O– bedeutet,

$R^7$ eine direkte Bindung, $C_1$–$C_{12}$ Alkylen, $C_2$–$C_6$ Alkenylen, $C_6$–$C_{12}$ Arylen darstellt,

$R^8$ und $R^9$ Wasserstoff oder $C_1$–$C_4$ Alkyl sind,

T' einen dreiwertigen Rest der Formeln

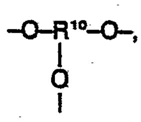,
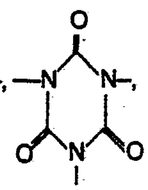,
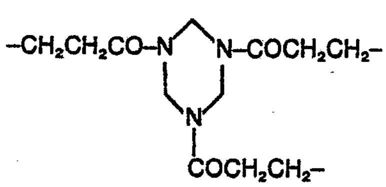

oder

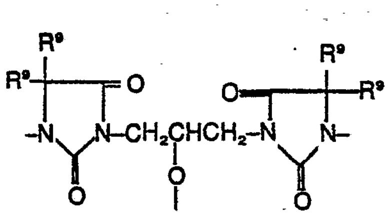

oder

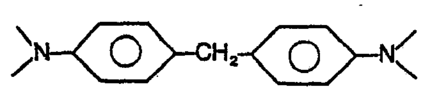

darstellt,

$R^{10}$ ein dreiwertiger aliphatischer Kohlenwasserstoffrest mit 3 bis 10 C-Atomen ist,

Q' einen vierwertigen Rest der Formel

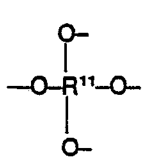

darstellt,

$R^{11}$ einen vierwertigen aliphatischen Kohlenwasserstoffrest mit 4 bis 10 C-Atomen bedeutet und

E und E' Endgruppen darstellen.

$R^1$, $R^2$, $R^3$ und $R^4$ als $C_1$–$C_{12}$ Alkyl können dabei verzweigte oder unverzweigte Alkylreste sein, z.B. Methyl, Äthyl, Isopropyl, tert.-Butyl, Hexyl, Isooctyl, Decyl oder Dodecyl.

$R^1$, $R^2$, $R^3$ als Alkoxyalkyl können z.B. Methoxymethyl, 2-Methoxyäthyl, 2-Äthoxyäthyl, 2-Isopropoxyäthyl, 2-n-, sec.- oder tert.-Butoxyäthyl oder 2-Butoxypropyl sein.

$R^1$, $R^2$, $R^3$ als Hydroxyalkyl können z.B. 2-Hydroxyäthyl, 2-Hydroxyäthyl, 2-Hydroxypropyl, 2-Hydroxybutyl oder 3-Hydroxypropyl sein.

$R^1$, $R^2$, $R^3$ als $C_5$–$C_{10}$ Cycloalkyl können beispielsweise Cyclopentyl, Cyclohexyl, 3-Methylcyclohexyl oder 4-tert.-Butylcyclohexyl sein.

$R^1$, $R^2$, $R^3$ als $C_6$–$C_{10}$ Aryl können Phenyl oder Naphthyl sein, wobei Phenyl bevorzugt ist.

$R^1$, $R^2$, $R^3$ als substituiertes Phenyl können z.B. p.-Tolyl, 4-Hydroxyphenyl, 4-tert.-Butylphenyl oder 3,5-Di-tert.-butyl-4-hydroxyphenyl sein.

X als $C_1$–$C_4$ Alkylen kann beispielsweise Methylen, Äthylen, 1,3-Propylen, 1,2-Propylen, 1,1-Dimethyläthylen oder 2,2-Propylen sein.

A, B oder $R^6$ als Alkylen kann eine verzweigte oder unverzweigte Alkylengruppe sein, wie z.B. Äthylen, Tri-, Tetra-, Hexa-, Octa-, Deca- oder Dodecamethylen, 2,2-Dimethylpropylen-1,3, 1,2-Butylen oder 1,2-Propylen. $R^5$ ist ein zweiwertiger aliphatischer oder cycloaliphatischer Rest, beispielsweise Äthylen, 1,2-Propylen, 1,2-Butylen, 1,3-Propylen, 1,4-Butylen, 1,6-Hexylen, 1,4-Cyclohexylen oder 3-Oxapentylen-1,5.

A und B als Alkenylen können z.B. 1,4-Buten-2-ylen oder 1,6-Hexen-3-ylen sein.

$R^6$ als Cycloalkylen kann z.B. 1,4-Cyclohexylen oder 1,4-Cyclooctylen sein. $R^6$ als Arylen kann Phenylen, Naphthylen oder Diphenylen sein.

$R^7$ als Alkylen oder Alkenylen kann z.B. Methylen, 1,3-Propylen, Tetramethylen, 2,2-Dimethyl-1,3-propylen, Octamethylen, Dodecamethylen, Vinylen oder 1,4-Buten-2-ylen sein. $R^7$ als cyclischer Rest kann beispielsweise 1,2-Cyclopentylen, 1,2-Cyclohexylen, 1,2-Cyclohexen-4-ylen, 3,6-Endomethylencyclohexen-4-ylen-1,2, 1,2-Phenylen, 1,4-Phenylen oder 1,4-Naphthylen sein.

Beispiele für Polyalkylpiperidin-Lichtschutzmittel dieser Klasse sind die Verbindungen der folgenden Formeln:

85)

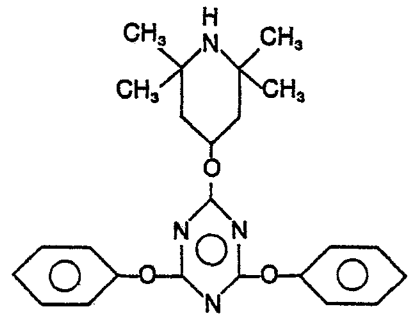

86)

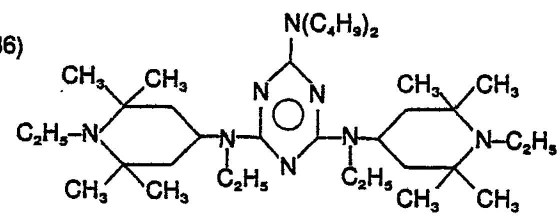

87)

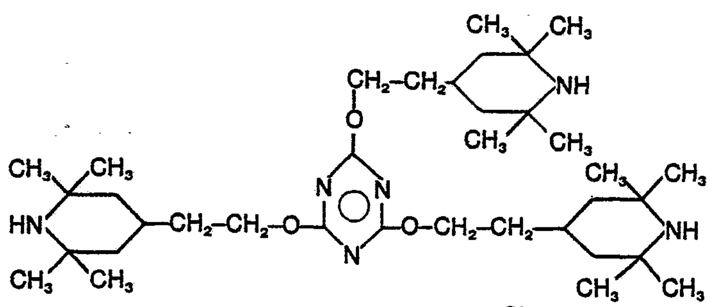

88)

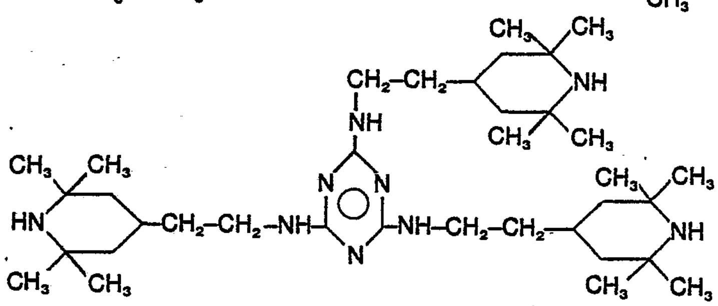

88a)

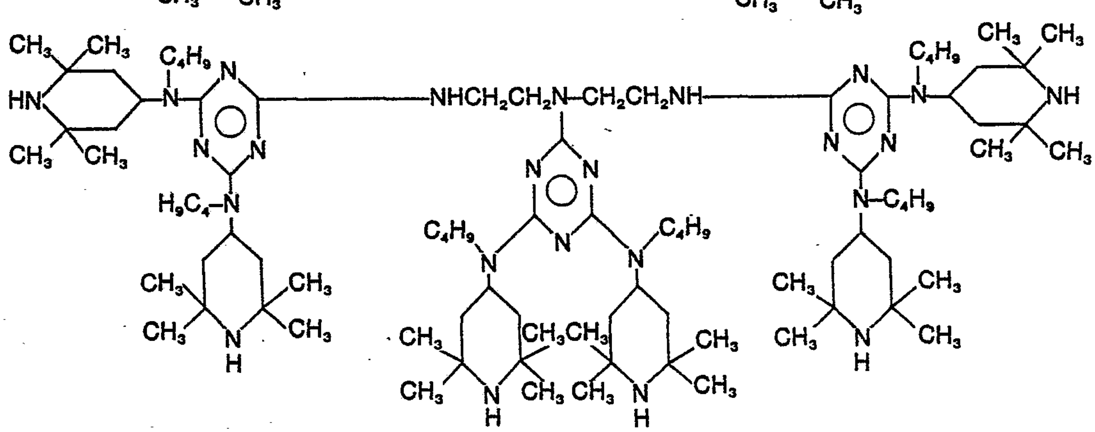

89) 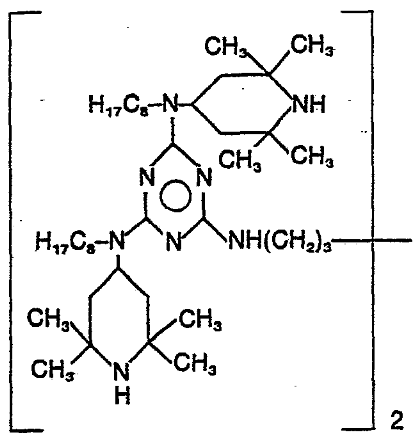

90) 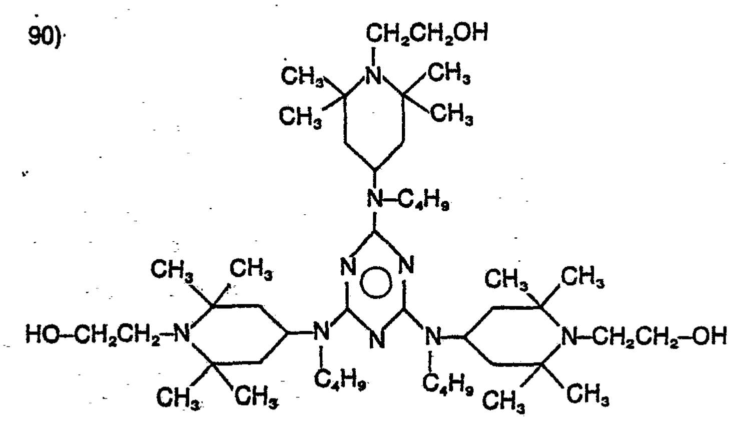

g) Lichtschutzmittel der Formel XVII

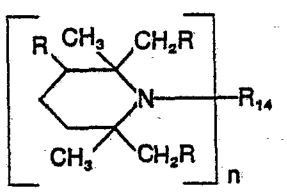 (XVII)

worin n die Zahlen 1 oder 2 bedeutet, R die für Formel VIII angegebene Bedeutung hat und $R_{14}$, wenn n = 1, $C_4$–$C_{18}$ Alkyl, $C_7$–$C_{12}$ Aralkyl, die Gruppe $-CO-R_{15}$, $C_1$–$C_4$ Alkyl, substituiert durch –CN, $-COOR_{16}$, –OH, $-OCOR_{17}$ oder

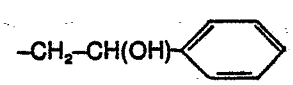

bedeutet, wobei $R_{15}$ $C_1$–$C_{12}$ Alkyl, $C_2$–$C_4$ Alkenyl oder Phenyl, $R_{16}$ $C_1$–$C_{18}$ Alkyl, $R_{17}$ $C_1$–$C_{18}$ Alkyl, $C_2$–$C_{10}$ Alkenyl, Cyclohexyl, Benzyl oder $C_6$–$C_{10}$ Aryl sind oder, wenn n = 2, $R_{14}$ $C_4$–$C_{12}$ Alkylen, 2-Butenylen-1,4, Xylylen, die Gruppe $-(CH_2)_2-OOC-R_{18}-COO-(CH_2)_2-$ oder die Gruppe $-CH_2-OOC-R_{19}-COO-CH_2-$ ist, wobei $R_{18}$ $C_2$–$C_{10}$ Alkylen, Phenylen oder Cyclohexylen und $R_{19}$ $C_2$–$C_{10}$ Alkylen, Xylylen oder Cyclohexylen bedeuten.

Bedeuten etwaige Substituenten $C_1$–$C_{12}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl oder n-Dodecyl dar.

Etwaige Substituenten, die $C_1$–$C_{18}$ Alkyl bedeuten, können z.B. die oben angeführten Gruppen und dazu noch beispielsweise n-Tridecyl, n-Tetradecyl, n-Hexadecyl oder n-Octadecyl darstellen.

Stellen etwaige Gruppen $C_2$–$C_{10}$ Alkylen dar, so bedeuten sie beispielsweise Äthylen, Propylen, 2,2-Dimethylpropylen, Tetramethylen, Hexamethylen, Octamethylen oder Decamethylen.

$R_{14}$ bedeutet als $C_4$–$C_{18}$ Alkyl z.B. n-Butyl, sek.-Butyl, tert.-Butyl, n-Hexyl, n-Octyl, 2-Äthyl-hexyl, 1,1-Dimethyl-2-tert.-butyläthyl, n-Nonyl, n-Decyl, n-Dodecyl, n-Tridecyl, n-Tetradecyl, n-Hexadecyl oder n-Octadecyl.

Bedeutet $R_{14}$ ein durch –CN substituiertes $C_1$–$C_4$ Alkyl, so stellt es beispielsweise Cyanomethyl, Cyanoäthyl, 3-Cyano-n-propyl, 4-Cyano-n-butyl dar.

Bedeutet $R_{14}$ $C_4$–$C_{12}$ Alkylen, so handelt es sich z.B. um 2,2-Dimethylpropylen, Tetramethylen, Hexamethylen, Octamethylen, Decamethylen oder Dodecamethylen.

Stellt $R_{14}$ $C_7$–$C_{12}$ Aralkyl dar, so bedeutet es insbesondere Phenäthyl, p-Methyl-benzyl oder vor allem Benzyl.

$R_{15}$ bedeutet als $C_2$–$C_4$ Alkenyl beispielsweise Vinyl, 1-Propenyl, Allyl, Methallyl, 2-Butenyl.

$R_{17}$ bedeutet als $C_2$–$C_{10}$ Alkenyl z.B. die für $R_{15}$ in der Bedeutung Alkenyl angeführten Gruppen und dazu noch beispielsweise Crotyl, 2-Hexenyl, 2-Octenyl oder 2-Decenyl.

Stellt $R_{17}$ $C_6$–$C_{10}$ Aryl dar, so bedeutet es beispielsweise gegebenenfalls in o- oder p-Stellung

mit Methyl, Äthyl, Isopropyl, n-Butyl oder tert.-Butyl substituiertes Phenyl.

Beispiele für Polyalkylpiperidin-Lichtschutzmittel dieser Klasse sind folgende Verbindungen:

91) Bis-[β-(2,2,6,6-tetramethylpiperidion)-äthyl]-sebacat

92) α-(2,2,6,6-Tetramethyl-piperidion)-essigsäure-n-octylester

93) 1,4-Bis-(2,2,6,6-tetramethylpiperidino)-2-buten.

h) Lichtschutzmittel der Formel XVIII

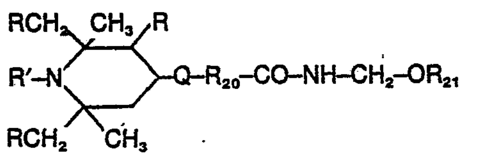

(XVIII)

worin Q $-N(R_{22})-$ oder $-O-$ ist, $R_{20}$ $C_1$–$C_3$ Alkylen, die Gruppe $-CH_2-CH(R_{23})-O-$, worin $R_{23}$ Wasserstoff, Methyl oder Phenyl ist, die Gruppe $-(CH_2)_3-NH-$ oder eine Einfachbindung bedeutet, R Wasserstoff oder Methyl ist, R′ die unter a) angegebene Bedeutung hat, $R_{21}$ Wasserstoff oder $C_1$–$C_{18}$ Alkyl bedeutet, $R_{22}$ Wasserstoff, $C_1$–$C_{18}$ Alkyl, $C_5$–$C_7$ Cycloalkyl, $C_7$–$C_{12}$ Aralkyl, Cyanäthyl, $C_6$–$C_{10}$ Aryl, die Gruppe $-CH_2-CH(R_{23})-OH$, worin $R_{23}$ die oben angegebene Bedeutung hat, eine Gruppe der Formel

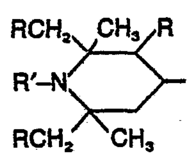

oder eine Gruppe der Formel

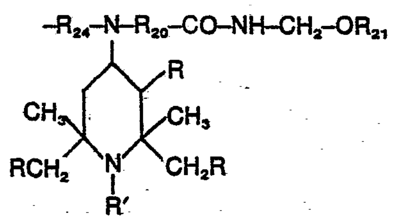

bedeutet, worin $R_2$ $C_2$–$C_{12}$ Arylen sein kann, oder $R_{22}$ eine Gruppe $-R_{20}-CO-NH-CH_2-OR_{21}$ ist.

Bedeuten etwaige Substituenten $C_1$–$C_{18}$ Alkyl, so stellen sie z. B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl, n-Dodecyl, n-Tridecyl, n-Tetradecyl, n-Hexadecyl oder n-Octadecyl dar.

Stellen etwaige Substituenten $C_7$–$C_{12}$ Aralkyl dar, so bedeuten sie beispielsweise Phenäthyl oder insbesondere Benzyl.

$R_{22}$ bedeutet als $C_5$–$C_7$ Cycloalkyl, insbesondere Cyclohexyl.

Als $C_6$–$C_{10}$ Aryl bedeutet $R_{22}$ insbesondere Phenyl α- oder β-Naphthyl, die gegebenenfalls mit Halogen oder $C_1$–$C_4$ Alkyl substituiert sind. $R_{20}$ bedeutet als $C_1$–$C_3$ Alkylen, z. B. Methylen, Äthylen oder Propylen.

$R_{24}$ stellt als $C_2$–$C_6$ Alkylen beispielsweise Äthylen, Propylen, 2,2-Dimethylpropylen, Tetramethylen oder Hexamethylen und als $C_6$–$C_{12}$ Arylen o-, m- oder p-Phenylen, 1,4-Naphthylen oder 4,4′-Diphenylen dar.

Beispiele für Polyalkylpiperidin-Lichtschutzmittel dieser Klasse sind folgende Verbindungen:

94) N-Hydroxymethyl-N′-2,2,6,6-tetramethyl-piperidin-4-yl-harnstoff

95) N-Methoxymethyl-N′-2,2,6,6-tetramethyl-piperidin-4-yl-harnstoff

96) N-Methoxymethyl-N′-n-dodecyl-N′-2,2,6,6-tetramethylpiperidin-4-yl-harnstoff

97) 0-(2,2,6,6-Tetramethylpiperidin-4-yl)-N-methoxy-methylurethan.

i) Polymere Verbindungen, deren wiederkehrende Struktureinheit einen Polyalkylpiperidinrest der Formel (VIII) enthält, insbesondere Polyester, Polyäther, Polyamide, Polyamine, Polyurethane, Polyharnstoffe, Polyaminotriazine, Poly(meth)-acrylate, Poly(meth)acrylamide und deren Copolymeren, die solche Reste enthalten.

98) Polyester aus Bernsteinsäure, Sebacinsäure, Di-butylmalonsäure, Oxalsäure oder Isophthalsäure und 1-Hydroxyäthyl-2,2,6,6-tetramethyl-4-hydroxypiperidin

99) Polyamid aus Bernsteinsäure, Adipinsäure oder Phthalsäure und 1-(3-Aminopropyl)-2,2,6,6-tetramethyl-4-aminopiperidin

100) Polyamid aus Oxalsäure oder p-Phenylendiessigsäure und N,N′-bis-(1,2,2,6,6-pentamethyl-4-piperidinyl)-1,6-diaminohexan

101) Polyamin aus 1,2,2,6,6-Pentamethyl-4-aminopiperidin und Epichlorhydrin oder Bisphenol-A-diglycidäther

102) Polytriazin aus 2,4-Dichlor-6-N-(1,2,2,6,6-pentamethyl-4-piperidinyl)-äthylamino-s-triazin und N,N′-Bis-(2,2,6,6-tetramethyl-4-piperidinyl)-1,6-diaminohexan

103) Copolymerisat aus 1-Benzyl-2,2,6,6-tetramethyl-4-acrylamidopiperidin und N-Butylacrylamid.

Beispiel für Polyalkylpiperidin-Lichtschutzmittel dieser Klasse sind die Verbindungen der folgenden Formeln, wobei m die Zahlen 2 bis 200 bedeutet.

104) 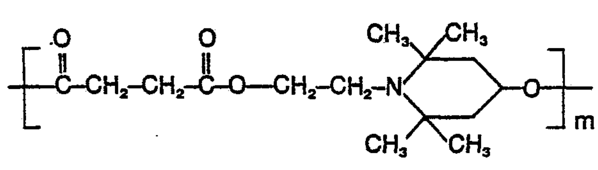

105) 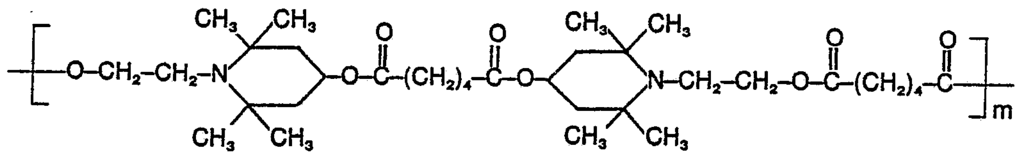

106 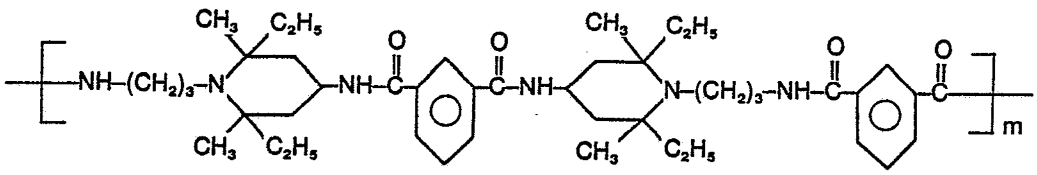

107) 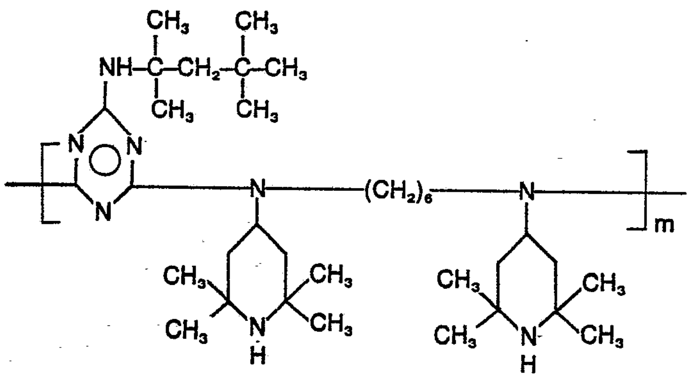

108) 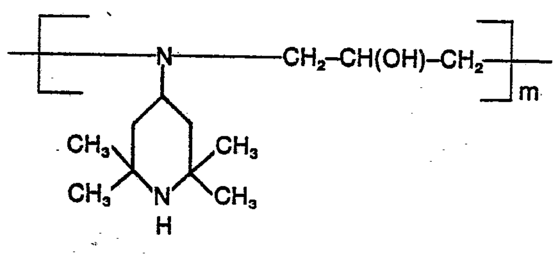

109) 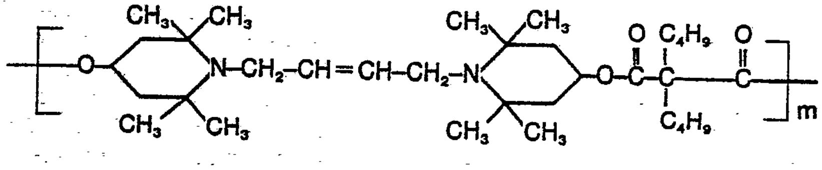

110) 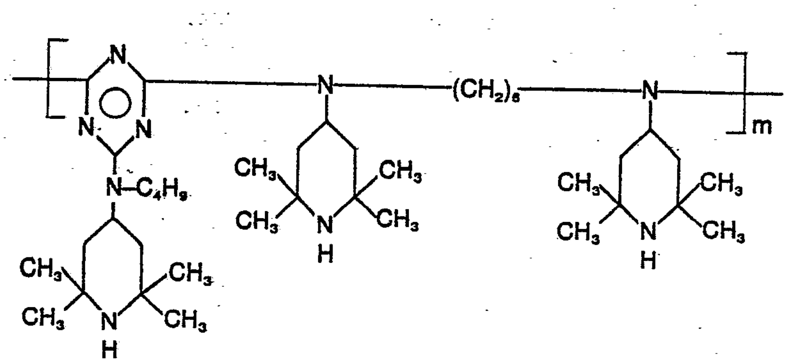

111) 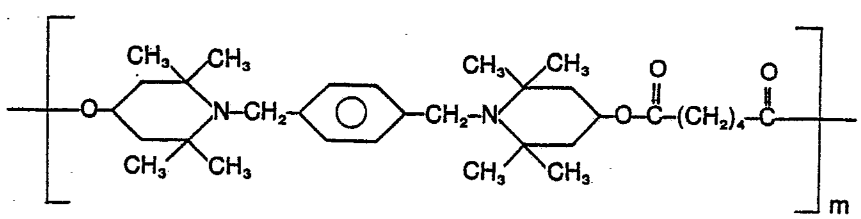

112) 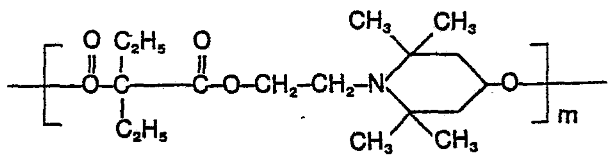

113) 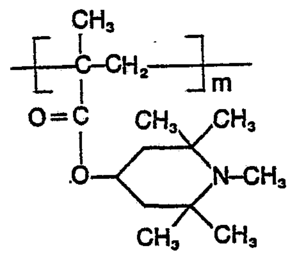

114) 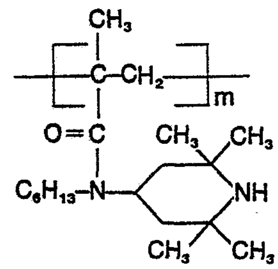

j) Verbindungen, die in ihrem Molekül mindestens eine 2-(2'-Hydroxyphenyl)-benztriazol-Gruppe oder 2-Hydroxybenzophenon-Gruppe und mindestens eine Polyalkylpiperidingruppe enthalten.

Beispiele für Polyalkylpiperidin-Lichtschutzmittel dieser Klasse sind die Verbindungen der folgenden Formeln

115) 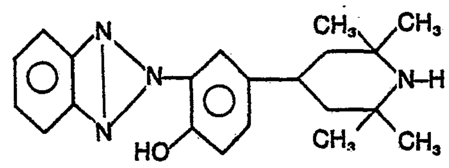

116) 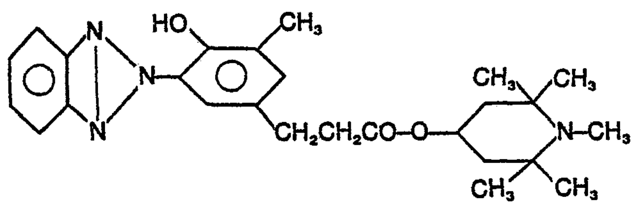

117) 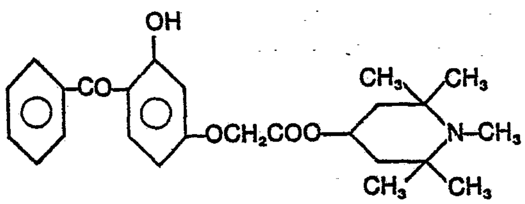

118) 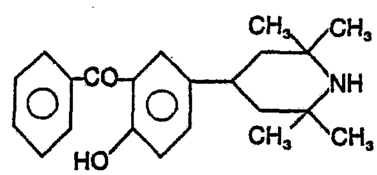

Es können auch Polyalkylpiperidinderivate der Klassen a) bis j) verwendet werden, die mit dem Bindemittel des Lackes chemische Bindungen eingehen. Dies ist der Fall, wenn das Polyalkylpiperidinderivat eine hierfür geeignete reaktive Gruppe besitzt, wie beispielsweise eine Glycidyl- oder eine Methylolgruppe.

Beispiele solcher Verbindungen sind die Methylol- bzw. Methyloläthergruppen enthaltende Polyalkylpiperidinderivate der Klasse h).

Soweit die Polyalkylpiperidinverbindungen basische Verbindungen sind, können sie mit Säuren Salze bilden. Hiefür kommen beispielsweise anorganische Säuren oder organische Carbon-, Sulfon-, Phosphon- oder Phosphinsäuren in Frage, wie z.B. Chlorwasserstoffsäure, Borsäure, Phosphorsäure, Essigsäure, Salicylsäure, Toluolsulfonsäure oder Benzolphosphonsäure.

Die Polyalkylpiperidinverbindungen können mit komplexbildenden Metallverbindungen Komplexe bilden, beispielsweise mit Zink-II-acetat, Cobalt-II-acetylacetonat, Nickel-II-acetylacetonat, Aluminium-III-acetylacetonat, Nickel-II-benzoat oder Aluminium-III-benzoylacetonat.

Salz aus 1 Mol $H_3PO_4$ und 1 Mol Adipinsäure-di-(1,2,2,6,6-pentamethyl-4-piperidinyl)-ester.

Salz aus 2 Mol Bis-(3,5-di-tert.-butyl-4-hydroxybenzyl)-malonsäure und 1 Mol 2,2,6,6-Tetramethyl-4-lauroyloxypiperidin.

1:1-Komplex von Bis-(2,2,6,6-tetramethyl-4-piperidinyl)-sebacat und Nickel-II-acetylacetonat.

1:2-Komplex von Bis-(1,2,2,6,6-pentamethyl-4-piperidinyl)-adipat und Nickel-II-acetat.

Die Verbindungen der Formel I verleihen gefärbten Polyamid- und Copolyamidfasern, wie z.B. Polyamid 6, Polyamid 6/6 oder Poly-m-phenylen-isophthalamid, ebenfalls ausgezeichnete Lichtstabilität.

Die Benzotriazole der Formel I unterscheiden sich in vier hervorragenden Eigenschaften von den strukturell nahestehenden Benzotriazolen des Standes der Technik. Sie sind

1. schwerflüchtig.

2. besser löslich in den Lösungsmitteln, welche für Kunststoffüberzüge üblicherweise verwendet werden.

3. vertragen sich besser mit Polyolefinen, Vinylpolymeren und Kohlenwasserstoffpolymeren.

4. widerstandsfähig gegenüber Metallionen, welche häufig in Stabilisator- und Härtersystemen für Polymere anzutreffen sind und zur Verfärbung vieler Benzotriazol-Stabilisatoren führen.

Die praktischen und wirtschaftlichen Vorteile, welche aus diesen unterwarteten Eigenschaften folgen, sind die folgenden:

a) Die Schwerflüchtigkeit, insbesondere wenn mit guter Substratverträglichkeit und Löslichkeit kombiniert, erlaubt die unproblematische Einführung der Benzotriazole der Formel I in das Polymer, wo sie auch nach der Hochtemperatur-Verarbeitung verbleiben und daher dem Endprodukt die gewünschte Stabilität verleihen. Wenn aber ein Stabilisator, sei er noch so wirksam, während der Verarbeitung verlorengeht, kann keine ausreichende stabilisierende Wirkung ausüben.

b) Wie aus der folgenden Tabelle ersichtlich ist, ist die Verbindung aus Beispiel 2, 2-(2-Hydroxy-3,5-di-tert.-octylphenyl)-2H-benzotriazol besser löslich in den Lösungsmitteln, welche für Kunststoffüberzüge üblicherweise verwendet werden als 2-(2-Hydroxy-3,5-di-tert.-amylphenyl)-2H-benzotriazol (TINUVIN 328®), welche die strukturell nächststehende Verbindung vom Stand der Technik ist.

Löslichkeit

| LM | Gramm/100 ml LM bei 20 °C<br>Verbindung aus Beispiel 2 | TINUVIN 328® |
|---|---|---|
| Xylol | 56 | 44 |
| Hexan | 35 | 16 |
| Äthylacetat | 35 | 16 |
| Styrol | 67 | 40 |

Die erhöhte Löslichkeit erlaubt die Verwendung der Benzotriazole der Formel I in konzentrierten Lösungen und damit die Arbeiten mit kleinen Lösungsmittelvolumen (was heute von besonderem Interesse ist für den Umweltschutz, aber auch interessant aus wirtschaftlichen Gründen). Ferner erlaubt es die Verwendung von weniger toxischen Lösungsmitteln, wie Hexan, an Stelle von aromatischen Lösungsmitteln.

c) Die grössere Substratverträglichkeit der Verbindungen der Formel I zusammen mit der bereits diskutierten Schwerflüchtigkeit gestattet die Verarbeitung und Verwendung der stabilisierten Produkte bei höheren Temperaturen sowie eine verlängerte Gebrauchsdauer der Produkte bei üblichen Temperaturen. Dadurch wird das unerwünschte Ausschwitzen des Stabilisators während der Verarbeitung verhindert, was sonst häufig zu kostspieligen Schädigungen der Anlage führt. Das Ausschwitzen des Stabilisators während des Gebrauchs der Faser oder des Kunststoffes, das sogenannte «blushing» wird ebenfalls vermieden. Das Ausschwitzen des Stabilisators ist einerseits unansehnlich, anderseits teuer, bedingt durch den frühzeitigen Verlust des Stabilisators und durch die verkürzte Gebrauchsdauer der Faser oder des Kunststoffes. Der Stabilisator geht verloren, noch bevor er seine Funktion hätte erfüllen können.

d) Die Verbindungen der Formel I sind überraschend resistent gegenüber Metallionen, welche in vielen Stabilisator- und Härtersystemen für Polymere vorkommen und zur Verfärbung führen. Viele bekannte Benzotriazol-Stabilisatoren formen offensichtlich farbige Metallkomplexe von unbekannter Struktur mit Ionen wie Kobalt, Eisen, Barium, Cadmium, Zinn usw. Solche Metallionen kommen z. B. in Polymeren, wie z. B. in ungesättigten Polyestern, Alkyden usw. vor, wenn man Kobaltnaphtenat zum Trocknen verwendet; in PVC oder PVC-Copolymeren, welche mit Barium/Cadmium oder mit Zinnverbindungen stabilisiert sind; in Polyurethanen, die Zinn-Katalysatoren enthalten; in ABS-Harzen, welche mit Eisen verunreinigt sind. In all diesen Substraten können die Benzotriazole der Formel I verwendet werden, dank ihrer Eigenschaft mit den erwähnten Metallionen keine Komplexe zu formen.

Dies wird durch die Gardner-Farbenzahl einer Xylol-Lösung demonstriert, welche verschiedene Benzotriazole enthält. Die Werte werden vor und nach Zugabe von zwei Tropfen 6%iger Kobaltnaphtenat-Lösung (in Xylol) gemessen.

Widerstandsfähigkeit gegen Verfärbung
Gardner-Farbenzahl*

| Stabilisator** | Vor Kobaltnaphtenat-Zugabe | Nach Kobaltnaphtenat-Zugabe |
|---|---|---|
| Keine | – | rötlich |
| Stabilisator 1 | 1 | schwach rötlich |
| TINUVIN 328® | 1 | 3,5 |
| TINUVIN P® | 1 | 10 |
| UV 5411® | 1 | 10 |

* Die Gardner-Farbenzahl 1 ist farblos (nicht gelb); 3,5 ist merklich gelb; 10 ist stark gelb.

** TINUVIN 328® = 2-(2-Hydroxy-3,5-di-tert.-amylphenyl)-2H-benzotriazol.
TINUVIN P® = 2-(2-Hydroxy-5-methylphenyl)-2H-benzotriazol.
UV 5411® = 2-(2-Hydroxy-5-tert.-octylphenyl-2H-benzotriazol.

Die Neigung mancher Benzotriazole, mit Metallionen Komplexe zu bilden, ist oft genug so nachteilig, dass man von ihrem Einsatz in metallhaltigen Systemen absehen muss. In anderen Fällen wiederum wäre die Verfärbung selber tolerierbar, aber die Komplexbildung ist nachteilig, indem sie verhindert, dass das Trockenmittel als Härter wirksam wird oder dass der Stabilisator seine Wirkung entfaltet. In all diesen Fällen ist von der Verwendung der bisher bekannten Benzotriazolen abzusehen.

e) Schliesslich in manchen hitzehärtbaren Acryl-Harzen, welche mit Melamin vernetzt werden, die Härtung durch die Gegenwart gewisser Benzotriazole verzögert. Es wurde nun gefunden, dass die Benzotriazole der Formel I, vermutlich infolge gewisser unerwarteter Konfiguration um die Hydroxyl-Gruppe herum, mit dem Melamin-Härter nicht reagieren und dadurch im Gegensatz zu manchen bekannten Benzotriazolen für hitzehärtbaren Acryl-Harze besonders geeignet sind.

Dies wird bestätigt, wenn man den Pendel-Härtewert (pendulum hardness value) des hitzehärtbaren Acrylharz-Filmes in Abwesenheit oder in Gegenwart von 1% Benzotriazol-Stabilisator bestimmt.

Pendel-Härtewert eines Acryl-Harzes*

| Stabilisator | Pendel-Härtewert** nach Härten (25 Minuten bei 140 °C) |
|---|---|
| Kein | 123 |
| TINUVIN 328® (1%)*** | 90 |
| Stabilisator 1 (1%) | 126 |

* Das Harz ist ein Styrol-substituiertes Acryl-Copolymer, das mit alkyliertem Melamin-Härter vernetzt wird.
** Je höher der Wert, desto härter der Überzug.
*** TINUVIN 328® = 2-(2-Hydroxy-3,5-di-tert.-amylphenyl)-2H-benzotriazol.

Auf unerklärbare Weise hindern noch beeinträchtigen die Benzotriazole der Formel I das Aushärten des Acrylharz-Systems, wie der beibehaltene oder sogar erhöhte Pendel-Härtewert es beweist. Der Pendel-Härtewert ist ein direktes Mass für den Grad der Aushärtung, und wie aus der Tabelle ersichtlich ist, steht der vom erfindungsgemässen Stabilisator gelieferte Wert im scharfen Kontrast zu dem Wert des strukturell nächststehenden Stabilisators.

Die folgenden Beispiele erläutern die Erfindung. Prozente sind Gewichtsprozente.

Beispiel 1
Herstellung des Zwischenproduktes 2-Nitro-2′-hydroxy-3′,5′-(di-tert.-octylphenyl)-azobenzol

In einen 5-Liter-Dreihals-Rundkolben mit Rührer und Thermometer gibt man 317,3 g einer 26%igen wässrigen Naphthalinsulfonsäure-Lösung, 6,7 g Triton X-207® (nichtionischer oberflächenaktiver Stoff), 19,6 g Conoco AAS-90F® (Natrium-dodecylbenzol-sulfonat) und 315 ml Wasser. Das Gemisch wird auf 40 °C erwärmt, dann mit 393,6 g 2,4-Di-tert.-octylphenol langsam versetzt und unter kräftigem Rühren auf 40 °C gehalten.

Eine kalte o-Nitrobenzoldiazoniumchlorid-Lösung wird aus 174,3 g (1,26 Mol) o-Nitroanilin und aus 87,1 g (1,26 Mol) Natriumnitrit in conc. HCl bei −5 °C bis 0 °C hergestellt. Diese Lösung wird tropfenweise während 1½ Stunden zum Reaktionsgemisch gegeben und die resultierende dunkelrote bis schwarze Lösung über Nacht bei 40 °C gehalten. Dann wird die Temperatur für 1 Stunde auf 65 °C und auf weitere 30 Minuten auf 95 °C erhöht. Nach Abkühlen auf 35 °C wird das Rohprodukt, ein tiefroter feinverteilter Feststoff, isoliert.

Das Rohprodukt wird mit 3,5 l Wasser zerrieben und anschliessend mit 1400 ml Methanol gerührt und abfiltriert. Man erhält 419,7 g (72,7% der Theorie) eines feinen Granulates. Smp. 110 bis 112 °C.

Das Dünnschichtchromatogramm zeigt ein homogenes Produkt.

Beispiel 2
2-(2-Hydroxy-3,5-di-tert.-octylphenyl)-2H-benzotriazol

In einen 5-Liter-Dreihals-Rundkolben mit Gaseinleitung, Rührer und Rückflusskühler gibt man 400 g (0,855 Mol) o-Nitroazobenzol, hergestellt im Beispiel 1, und 1200 ml Toluol. Zu dieser Lösung fügt man 260 ml Isopropanol und 260 ml Wasser. Unter Einleiten von Stickstoff fügt man 175 ml 50,1%ige Natronlauge zur Lösung. Eine Flasche mit 170,0 g (2,6 Grammatom) Zink wird an den Reaktionskolben angeschlossen. Man leitet den Zinkstaub portionsweise während 120 Minuten in das Reaktionsgemisch, und zwar so, dass die Reaktionstemperatur zwischen 70 °C bleibt. Nachdem alles Zink zugefügt wurde, gibt man weitere 30 ml 50,1%ige Natronlauge und 20 g Zink dazu, um eine vollständige Reaktion zu sichern. Nach Beendigung der Zinkzugabe erwärmt man das Reaktionsgemisch auf 70 °C 3 Stunden lang. Das Gemisch wird auf Raumtemperatur abgekühlt und mit 500 ml konz. Salzsäure angesäuert.

Der Zink-Rückstand wird durch Filtrieren entfernt. Die organische Phase, welche das Produkt enthält, wird dreimal mit 1000 ml Wasser, dann mit 500 ml gesättigter Salzlösung gewaschen und über wasserfreiem Natriumsulfat getrocknet. Nach Entfernen des Lösungsmittels durch Vakuumdestillation erhält man das viskose Rohprodukt, das beim Stehen kristallisiert.

Um das Rohprodukt zu reinigen, rekristallisiert man es zweimal aus 1000 bis 1100 ml Äthanol.

Man erhält 253 g (67,8% der Theorie) blassgelbe Kristalle. (Stabilisator 1) Smp. 105 bis 106 °C.

Analyse: $C_{28}H_{41}N_3O$:

| | | | |
|---|---|---|---|
| berechnet: | C 77,20 | H 9,49 | N 9,65 |
| gefunden: | C 77,22 | H 9,14 | N 9,77 |

Beispiel 3
4-Chloro-2-nitro-2'-hydroxy-3',5'-di-tert.-octylazobenzol

Wenn man im Beispiel 1 anstelle der Diazoniumlösung, hergestellt aus 2-Nitroanilin, eine Diazoniumlösung, hergestellt aus 4-Chloro-2-nitroanilin in äquivalenter Menge verwendet, erhält man 61,6% vom dunkelroten Produkt.

Beispiel 4
5-Chloro-2-(2'-hydroxy-3',5'-di-tert.-octylphenyl)-2H-benzotriazol

Wenn man im Beispiel 2 anstelle von 2-Nitro-2'-hydroxy-3',5'-di-tert.-octyl-azobenzol 4-Chloro-2-nitro-2'-hydroxy-3',5'-di-tert.-octyl-azobenzol verwendet, erhält man 71,8% vom Produkt. Die blassgelben Kristalle schmelzen bei 121 bis 122 °C. (Stabilisator 2)

Analyse: $C_{28}H_{40}ClN_3O$:

| | | | |
|---|---|---|---|
| berechnet: | C 71,54 | H 8,58 | N 8,94 |
| gefunden: | C 71,26 | H 8,46 | N 9,03 |

Beispiel 5
Verlustwiderstand des Benzotriazols

Eine Anzahl von 2-Aryl-2H-benzotriazolen werden thermalgravimetrisch analysiert, und zwar sowohl isothermisch bei 280 °C, um die Zeit in Minuten zu ermitteln, bei welcher 10, 50 und 100% des Stabilisators verlorengeht, als auch durch eine Auszähl-Methode, bei der die Temperatur kontinuierlich um 10 °C pro Minute erhöht wurde, bis ein Stabilisatorschwund von 10 bzw. 50% festgestellt wurde.

Die Testergebnisse sind in der Tabelle A zusammengefasst.

Die Ergebnisse zeigen das genaue Mass an Stabilisatorwiderstand während der Verarbeitung des Polymeres zu Folien, Filmen, Fasern usw., nicht auszuschwitzen oder zu verflüchtigen. Dadurch, dass kein Stabilisator infolge Verflüchtigen oder Ausschwitzen sich auf die Anlage niederschlägt, ist die Zeit bis zur Schädigung der Anlage wesentlich erhöht. Daher ist die Verwendung der Stabilisatoren der Formel I sowohl von praktischer als auch von wirtschaftlicher Bedeutung.

Tabelle A

| Stabilisator* | Thermalgravimetrische Analysedaten isometrisch bei 280 °C Zeit in Minuten bis zum Gewichtsverlust von | | | Temperatur in °C bei einer Temperatur-Steigerung von 10 °C/Minute bis zum Gewichtsverlust von | |
|---|---|---|---|---|---|
| | 10% | 50% | 100% | 10% | 50% |
| TINUVIN P® | 0,4 | 0,75 | 1,2 | 182 | 215 |
| TINUVIN 350® | 0,6 | 1,0 | 1,8 | 210 | 247 |
| CYASORB UV-5411® | 0,6 | 1,9 | 3,5 | 225 | 260 |
| Stabilisator 1 | 1,0 | 3,2 | 6,0 | 240 | 280 |

* TINUVIN P® ist 2-(2-Hydroxy-5-methylphenyl)-2H-benzotriazol.
* TINUVIN 350® ist 2-(2-Hydroxy-3-tert.-butyl-5-sec-butylphenyl)-2H-benzotriazol.
CYASORB UV-5411® ist 2-(2-Hydroxy-5-tert.-octylphenyl)-2H-benzotriazol.

Der Stabilisator 1 (aus Beispiel 2) ist eindeutig schwerflüchtiger als die anderen Benzotriazole. Der Stabilisator 1, eingearbeitet in das Polymer, würde daher dem Polymer einen besseren Schutz gegen schädliche Lichteinwirkungen bieten und darüber hinaus ein ausgezeichnetes Verhalten während der Verarbeitung zeigen.

Beispiel 6
Beständigkeit von Benzotriazol-Stabilisatoren in Polycarbonat während der Folienherstellung.

Polycarbonatharz (Lexan®, General Electric) wurde mit 0,3% (Gewicht) diverser 2-Aryl-2H-benzotriazol-UV-Absorber ausgerüstet. Das formulierte Harz wurde bei 316 °C zu dünnen Folien extrudiert. Die erhaltenen Folien wurden in Methylenchlorid gelöst und das Polycarbonat mit Methanol ausgefällt. Die Menge an Benzotriazol-Stabilisator, die in der Polycarbonat-Folie nach der Herstellung verblieb, wurde mittels Gaschromatographie bestimmt.

Die Ergebnisse zeigt Tabelle B.

Tabelle B

| Stabilisator* | % verblieben in der Polycarbonat-Folie nach Herstellung |
|---|---|
| TINUVIN 350® | 82 |
| CYASORB UV-5411® | 87 |
| Stabilisator 1 | 100 |

* siehe Tabelle A für die chemischen Namen dieser Stabilisatoren.

Diese Daten bestätigen die Ergebnisse von Tabelle A, nämlich die geringere Flüchtigkeit des Stabilisators 1 während der Verarbeitung.

Beispiel 7
Beständigkeit gegen Verluste beim Härten und Bewittern von Benzotriazol-Stabilisatoren in Thermoset-Acrylüberzügen

Diverse Thermoset-Acrylharze und ein Alkyd/Acrylharz-System wurden mit 2% (Gewicht) verschiedener Benzotriazol-UV-Absorber formuliert und auf Glasplatten zu 1 µ dicken Überzügen gegossen.

Die Überzüge wurden dann bei erhöhter Temperatur eine bestimmte Zeit gehärtet. Der Verlust an Benzotriazol-UV-Absorber wurde dann durch UV-Absorptions-Analyse der Überzüge bestimmt. Eine Abnahme der Absorption der Überzüge kann einem Verlust an Benzotriazol-Stabilisator während des Härtens gleichgesetzt werden.

Diese gehärteten Überzüge wurden dem beschleunigten (quick) Bewitterungstest (QUV) unterworfen, bei dem alternierend 4-Stunden-UV-Bestrahlung bei 60 °C und 4-Stunden-Kondensation (Regen) bei 50 °C über eine Zeitspanne von 670 Stunden einwirken. Wiederum kann eine Absorptionsabnahme der bewitterten Überzüge mit einem Verlust an Benzotriazol-Stabilisator während des Härtens und der Bewitterung gleichgesetzt werden. Die Ergebnisse zeigt Tabelle C.

Tabelle C Absorptionsverlust während Härten oder Bewitterung (%)

| Stabilisator* | Thermoset-Acrylharz-Systeme | | | | | Alkyd/Acrylharz | |
|---|---|---|---|---|---|---|---|
| | Einfache Schicht | | 2-Schicht-System | | High-Solids | | |
| | erhitzt 25 Min. bei 120 °C | nach Bewitterung | erhitzt 20 Min. bei 135 °C | nach Bewitterung | erhitzt 30 Min. bei 150 °C | erhitzt 30 Min. bei 125 °C | Nach Bewitterung |
| TINUVIN 328® | 25 | 35 | 59 | 69 | 95 | 77 | 100 |
| Stabilisator 1 | 6 | 21 | 24 | 41 | 67 | 27 | 80 |

* TINUVIN 328® ist 2-(2-Hydroxy-3,5-di-tert.-amylphenyl)-2H-benzotriazol.

Tabelle C zeigt, dass die Verbindung der Formel I viel weniger Verluste in den Thermoset-Acryl-Harz- und Alkyd/Acrylharz-Systemen erleidet als bekannte Benzotriazole. Stabilisator 1 ist beträchtlich weniger flüchtig als TINUVIN 328®, das eine vergleichbare Struktur hat.

Beispiel 8

Stabilisation von Polyäthylen-terephthalat

0,5% der Verbindung von Beispiel 2 wird als Stabilisator zu geschmolzenen Polyäthylen-teraphthalat bei 270 °C unter Rühren und einer Stickstoffatmosphäre gegeben. Der erhaltene, formulierte Polymer wird mit festem Kohlendioxid gemahlen.

Die stabilisierte Mischung wird bei erhöhter Temperatur zu einem Film unter geringem Stabilisatorverlust extrudiert. Dann wird der Film einer UV-Bestrahlung unterworfen. Der stabilisierte Film behält seine erwünschten physikalischen Eigenschaften länger als der unstabilisierte.

Beispiel 9

Stabilisation von Polycarbonat

Polycarbonat (Lexan®, General Electric) wird in einem Extruder mit 0,3% der Verbindung von Beispiel 4 gemischt. Die stabilisierte Mischung wird extrudiert zu einer Folie bei erhöhter Temperatur unter geringem Verlust an Stabilisator. Die Folie behält ihre physikalischen Eigenschaften nach UV-Belichtung länger als eine solche ohne Stabilisator.

Die Delamination von UV-transparenten Automobil-Decklacken, aufgetragen auf Epoxy-Ester-Primer-Oberflächen ist ein schwerwiegendes Problem der Automobilhersteller. Dieses Problem wird besonders relevant, wenn die Decklack-Stärke unter der Vorschrift liegt. Einarbeiten von erfindungsgemässen UV-Absorbern in den Decklack schützt diesen vor Delamination und vor Glanzverlust.

Beispiel 10

Glanz- und Delaminations-Werte von Decklacken aus thermoplastischem Acrylat

Ein silbermetallic-thermoplastischer Acryllack wurde formuliert mit einem Benzotriazol-UV-Absorber und dann als Decklack auf eine Primer-Oberfläche aus einem Epoxy-Ester auf Metallplatten gesprüht. Das Ganze wurde 10 Minuten bei 48 °C und dann 30 Minuten bei 155 °C erhitzt. Die ursprüngliche Decklackschichtdicke war 2,0 bis 2,2 mils (50 bis 55 Micron, 0,0508 bis 0,0559 mm).

Die Platten wurden ein Jahr in Süd-Florida in einem ungeheizten schwarzen Kasten bei einem Winkel von 5° zur Sonne belichtet.

Die belichteten Platten wurden 96 Stunden in eine Kammer mit konstanter Feuchtigkeit bei 38 °C und 100% relativer Feuchte gegeben. Dann wurden sie abgetrocknet und sofort begutachtet, mittels des «cross-hatch tape»-Adhäsionstests. Dann wurden die Platten 1 Stunde bei Raumtemperatur belassen, bevor der «cross-hatch tape»-Adhäsionstest an einer andern Stelle der selben Platte wiederholt wurde. Die Proben zeigen allgemein eine etwas verbesserte Delaminationsbeständigkeit nach dieser 1stündigen Erholungspause, gegenüber der Delamination sofort nach der Feuchtigkeitsbehandlung.

Beim «cross-hatch tape»-Adhäsionstest wird ein Mehrschneidemesser benutzt, um Schnitte durch den Decklack auf der Platte anzubringen. Ein Acetatfaser-Klebeband wird über die Schnittstellen geklebt und dann abgezogen. Der Decklack wird visuell beurteilt, ob und wieviel abgezogen wurde. Dies ergibt eine relative Beurteilung der Delamination von 0 (keine «cross-hatch»-Delamination) bis 5 (vollständige «cross-hatch»-Delamination). Die Ergebnisse zeigen Tabellen D und E.

Tabelle D

| Stabilisatoren (% Gewicht)** | 20°-Glanzwerte von silbermetallic thermoplastischem Acryllack* nach 5°-Süd-Florida-Black-Box-Belichtung vorher | nach 6 Monaten (Florida) | nach 1 Jahr (Florida) | nach 1 Jahr poliert*** |
|---|---|---|---|---|
| keiner | 77,8 | 15,1 | 1,6 | 4,4 |
| 1% Stabilisator 1 | 77,7 | 38,0 | 2,6 | 7,7 |
| 2% TINUVIN 328® | 77,1 | 34,9 | 8,5 | 13,7 |
| 2% TINUVIN 350® | 77,5 | 37,5 | 6,0 | 24,3 |
| 2% Stabilisator 1 | 77,5 | 35,4 | 7,1 | 14,7 |
| 2% Stabilisator 2 | 78,3 | 36,7 | 3,5 | 9,4 |

* Thermoplastischer Acryllack besteht aus einem Binder von 60% Poly(methylmethacrylat, 20% Celluloseacetatbutyrat und 20% Weichmacher, zusammen mit etwa 3% metallischem Pigment.

** Siehe Tabellen A und C für chemische Namen dieser Stabilisatoren.

*** Eine Hälfte jeder Platte wurde mit DuPont Nr. 7 Autopoliermittel poliert.

Tabelle E

Delaminationswerte für silbermetallic-thermoplastischen Acryllack* nach einem Jahr 5°-Süd-Florida-Black-Box-Belichtung

| Stabilisator** (% Gewicht) | Delaminationswert (0 bis 5) Sofort nach Feuchtigkeitsbehandlung | Nach einer Stunde Erholung bei Raumtemperatur nach Feuchtigkeitsbehandlung |
|---|---|---|
| keiner | 5 | 5 |
| 1% Stabilisator 1 | 5 | 3 |
| 2% TINUVIN 328® | 5 | 2 |
| 2% TINUVIN 350® | 3 | 2 |
| 2% Stabilisator 1 | 5 | 3 |
| 2% Stabilisator 2 | 3 | 1 |

* Siehe Beschreibung des Lacks zu Tabelle D.

** Siehe Tabelle A und C für chemische Namen dieser Stabilisatoren.

Die Daten der Tabelle D bestätigen, dass der Stabilisator 1 und 2 ausgezeichneten Schutz gegen Verluste während längerer Belichtung im Süd-Florida-Black-Box-Test bewirken, verglichen mit anderen Benzotriazol-Lichtschutzmitteln.

Die Daten der Tabelle E zeigen, dass die erfindungsgemässen Verbindungen, insbesondere Stabilisator 2, einen thermoplastischen Acryl-Decklack vor Delamination nach 1 Jahr Süd-Florida-Black-Box-Belichtung unter strengsten Testbedingungen mit sofort nachfolgender Feuchtigkeitsbehandlung schützen.

Beispiel 11

Delaminationswerte von Decklacken aus Thermoset-Acryl-Emaille

Zwei silbermetallic-Thermoset-Acryl-Emailles wurden mit einem Benzotriazol-Lichtschutzmittel formuliert und dann als Decklack auf eine Primeroberfläche aus einem Epoxy-Ester auf einer Metallplatte gesprüht. 17 Minuten lang wurde dieser bei 130 °C zu einem Anfangsdecklackfilm von 1,7 mil Dicke (42 Micron, 0,0432 mm) gehärtet. Die Platte wurde 1200 Stunden in dem QUV-Bewitterungstest gemäss Beispiel 10 behandelt.

Nach QUV-Belichtung wurden die Platten feuchtigkeitsbehandelt und dann die Delamination bestimmt, dann 1 Stunde Erholung eingeschaltet, worauf erneut die Delaminationsbeständigkeit gemäss Beispiel 10 bestimmt wurde. Die Ergebnisse zeigt Tabelle F.

Tabelle F

Delaminationswerte einer silbermetallic Thermoset-Acryl-Emaille nach 1200 Stunden QUV-Belichtung

| Stabilisator (% Gewicht) | Delaminationswert (0 bis 5) Emaille * A Sofort nach Feuchtigkeitsbehandlung | Emaille * A Nach 1 Std. Erholung bei Zimmertemperatur nach Feuchtigkeitsbehandlung | Emaille * B Sofort nach Feuchtigkeitsbehandlung | Emaille * B Nach 1 Std. Erholung bei Zimmertemperatur nach Feuchtigkeitsbehandlung |
|---|---|---|---|---|
| keiner | 3 | 1 | 5 | 3 |
| 1% Stabilisator 1 | 1 | 1 | 0 | 0 |
| 2% Stabilisator 1 | 0 | 0 | 0–1 | 0 |

* Thermoset-Acryl-Emaille, bestehend aus einem Binder von 70% Acrylmonomeren wie Hydroxyäthyl-acrylat, Styrol, Acrylnitril, Butylacrylat und Acrylsäure mit 30% eines Melaminharzes. Emaille A ist eine Nieder(40–45%)-Fertig-Lösung. Emaille B ist eine nichtwässrige Dispersion (NAD) mit etwa 45–50% Feststoff und etwa 3% metallischem Pigment.

Die Kombination von sterisch gehinderten Amin-Lichtschutzmitteln mit den erfindungsgemässen Benztriazol-UV-Absorbern ist besonders geeignet, den Glanz zu erhalten und Delamination zu verhindern, insbesondere bei metallisierten Thermoset-Acryl-Emaillen und bei metallisierten thermoplastischen Acryllacken für Automobildecklacke.

Die gehinderten Amine schützen die Thermoset-Acryl-Emaille und den thermoplastischen Acryllack selbst bei niedriger Konzentration gegen Glanzverluste, wirken aber nicht als UV-Filter. Demgemäss kann UV-Licht durch den Acryldecklack treten, falls kein UV-Absorber vorhanden ist, wie die erfindungsgemässen Benztriazole, und kann Schaden anrichten in der Epoxy-Ester-Primer-Oberfläche unter dem Decklack. Einarbeiten von selbst niedrigen Konzentrationen (0,5% Gewicht) eines Benztriazols in Kombination mit einem gehinderten Amin bewirkt sowohl Glanzerhaltung als auch Widerstandsfähigkeit gegen Delamination für metallisierte Acryl-Decklacke.

Beispiel 12

Delaminationswerte für Decklacke aus Thermoset-Acryl-Emaille

Zwei silbermetallisierte Thermoset-Acryl-Emaillen wurden formuliert sowohl mit einem gehinderten Amin-Lichtschutzmittel als auch einem Benzotriazol-UV-Absorber. Die Testplatten wurden wie im Beispiel 11 beschrieben hergestellt und getestet.

Wird der Thermoset-Acryl-Emaille nur mit einem sterisch gehinderten Amin kombiniert, wie z.B. Bis-(2,2,6,6-tetramethyl-4-piperidyl)-sebacat oder Bis-(1,2,2,6,6-pentamethyl-4-piperidyl)-2-n-butyl-2-(3,5-di-tert.-butyl-4-hydroxybenzyl)-malonat, so sind die Delaminationswerte identisch mit den Werten des unstabilisierten Emaille. Ausgezeichnete Delaminationswerte erhält man durch die Kombination von sterisch gehinderten Amin-Lichtstabilisatoren und der erfindungsgemässen Benzotriazol-UV-Absorber in den Emaillen.

Beispiel 13

Delamination und 20°-Glanzwerte von thermoplastischen Acryl-Lacken

Die Wirksamkeit von Kombinationen von gehinderten Amin-Lichtschutzmitteln mit erfindungsgemässen Benztriazol-UV-Absorbern zum Schutz von Automobildecklacken zeigt sich bei thermoplastischen Acrylharzen, wobei sowohl Glanzerhalt nach längerer Belichtung in Süd-Florida durch das gehinderte Amin erfolgt, als auch durch die erfindungsgemässen Benztriazol-UV-Absorber Schutz des thermoplastischen Acryl-Decklackes vor Delamination. Ein silbermetallic thermoplastischer Acryl-Lack wurde formuliert sowohl mit einem gehinderten Amin-Lichtschutzmittel, als auch einem Benztriazol-UV-Absorber. Testplatten wurden hergestellt und getestet wie in Beispiel 10 beschrieben.

Der Lack, welcher sowohl ein Amin-Lichtschutzmittel (wie z.B. im Beispiel 12 beschrieben) als auch einen Benzotriazol-Stabilisator enthielt (z.B. Stabilisator 1 oder 2), zeigte ausgezeichnete Glanz- und Delaminationswerte bei 20 °C.

Beispiel 14

Die Kombination von gewissen Benzotriazol-UV-Absorbern mit gewöhnlichen Barium/Cadmium-Thermostabilisatoren für Polyvinylchlorid (PVC) führt zur Verfärbung. Diese Verfärbung beschränkt daher die Brauchbarkeit dieser Benzotriazole in gewissen PVC-Applikationen.

Die gegenwärtigen Benzotriazole (Stabilisator 1) zeigten sich wesentlich widerstandsfähiger gegenüber Verfärbung in Gegenwart von solchen PVC-Thermostabilisatoren als z.B. 2-Hydroxy-5-methylphenyl-2H-benzotriazol.

**Patentansprüche**

1. Stabilisiertes organisches Polymer, enthaltend als Lichtstabilisator eine Verbindung der Formel I

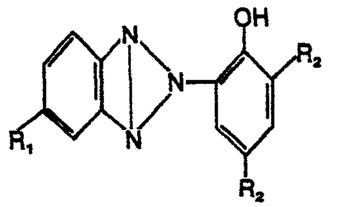

(I),

worin $R_1$ Wasserstoff oder Chlor ist und $R_2$ t-Octyl bedeutet.

2. Stabilisiertes organisches Polymer gemäss Anspruch 1, dadurch gekennzeichnet, dass der Stabilisator 2-[2-Hydroxy-3,5-di-tert.-octylphenyl]-2H-benzotriazol ist.

3. Stabilisiertes organisches Polymer, enthaltend 0,1 bis 5 Gew.-% eines Stabilisators gemäss Anspruch 1.

4. Stabilisiertes organisches Polymer gemäss Anspruch 1, dadurch gekennzeichnet, dass das Material ein synthetisches Polymer ist.

5. Stabilisiertes Polymer gemäss Anspruch 1, dadurch gekennzeichnet, dass das Polymer ein Polyester, Polycarbonat, heissvernetzbares Acrylharz, Polyamid oder ein Polyurethan ist.

6. Lack mit Glanzhaltung und Abblätterungsbeständigkeit bei Bewitterung, enthaltend

a) heissvernetzbares Acrylharz und

b) 0,1 bis 5 Gew.-%, bezogen auf das Harz, einer Verbindung der Formel I gemäss Anspruch 1, und

c) 0,1 bis 5 Gew.-%, bezogen auf das Harz, eines sterisch gehinderten Amin-Stabilisators.

7. Lack gemäss Anspruch 6, enthaltend 0,5 bis 2 Gew.-% von b) und 0,5 bis 2 Gew.-% von c).

8. Lack gemäss Anspruch 6, enthaltend als Bestandteil c) Bis-(2,2,6,6-tetramethyl-4-piperidyl)-sebacat als Lichtschutzmittel.

9. Lack gemäss Anspruch 6, enthaltend als Bestandteil c) Bis-(1,2,2,6,6-pentamethyl-4-piperidyl)-2-n-butyl-2-(3,5-di-tert.-butyl-4-hydroxybenzyl)-malonat als Lichtschutzmittel.

**Claims**

1. A stabilised organic polymer containing, as light stabiliser, a compound of the formula

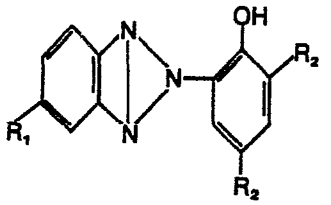

(I),

wherein $R_1$ is hydrogen or chlorine, and $R_2$ is tert-octyl.

2. A stabilised organic polymer according to claim 1, which contains 2-[2-hydroxy-3,5-di-tert-octylphenyl]-2H-benzotriazole as light stabiliser.

3. A stabilised organic polymer which contains 0.1 to 5% by weight of a stabiliser as claimed in claim 1.

4. A stabilised organic polymer according to claim 1, wherein the material is a synthetic polymer.

5. A stabilised polymer according to claim 1, wherein the polymer is a polyester, a polycarbonate, a thermoset acrylic resin, a polyamide or a polyurethane.

6. A lacquer which retains its gloss and is resistant to peeling, which lacquers contains

a) a thermoset acrylic resin and

b) 0.1 to 5% by weight, based on said resin, of a compound of the formula I according to claim 1, and

c) 0.1 to 5% by weight, based on said resin, of a sterically hindered amine stabiliser.

7. A lacquer according to claim 6, which contains 0.5 to 2% by weight of b) and 0.5 to 2% by weight of c).

8. A lacquer according to claim 6, which contains bis-(2,2,6,6-tetramethyl-4-piperidyl) sebacate as light stabiliser c).

9. A lacquer according to the claim 6, which contains bis-(1,2,2,6,6-pentamethyl-4-piperidyl)-2-n-butyl-2-(3,5-di-tert-butyl-4-hydroxybenzyl)-malonate as light stabiliser c).

**Revendications**

1. Polymère organique stabilisé contenant, comme stabilisant à la lumière, un composé répondant à la formule

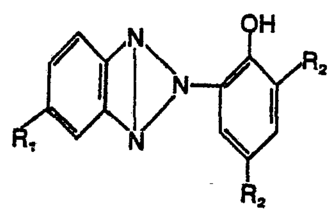

(I),

dans laquelle $R_1$ représente l'hydrogène ou le chlore et $R_2$ représente un radical tert-octyle.

2. Polymère organique stabilisé selon la revendication 1, caractérisé en ce que le stabilisant est l'(hydroxy-2 di-tert-octyl-3,5 phényl)-2 2H-benzotriazole.

3. Polymère organique stabilisé contenant de 0,1 à 5% en poids d'un stabilisant selon la revendication 1.

4. Polymère organique stabilisé selon la revendication 1, caractérisé en ce que le polymère est un polymère synthétique.

5. Polymère stabilisé selon la revendication 1, caractérisé en ce que le polymère est un polyester, un polycarbonate, une résine acrylique réticulable par la chaleur, un polyamide ou un polyuréthanne.

6. Vernis conservant bien son brillant et ayant une bonne résistance à l'écaillage sous l'action des agents atmosphériques, vernis qui contient:

a) une résine acrylique réticulable par la chaleur,

b) de 0,1 à 5% en poids, par rapport à la résine, d'un composé de formule I selon la revendication 1, et

c) de 0,1 à 5% en poids, par rapport à la résine, d'un stabilisant qui est une amine à empêchement stérique.

7. Vernis selon la revendication 6 qui contient de 0,5 à 2% en poids de b) et de 0,5 à 2% en poids de c).

8. Vernis selon la revendication 6 qui contient, comme constituant c), du sébaçate de bis-(tétraméthyl-2,2,6,6 pipéridyle-4) en tant que stabilisant à la lumière.

9. Vernis selon la revendication 6 qui contient, comme constituant c), du n-butyl-2 (di-tert-butyl-3,5 hydroxy-4 benzyl)-2 malonate de bis-(pentaméthyl-1,2,2,6,6 pipéridyle-4) en tant que stabilisant à la lumière.